# EUROPÄISCHE PATENTSCHRIFT

(11) **EP 3 204 105 B1**
(45) Veröffentlichungstag und Bekanntmachung des Hinweises auf die Patenterteilung: **01.08.2018**
(21) Anmeldenummer: 15774937.5
(22) Anmeldetag: 07.10.2015
(51) Int. Cl.: A61N 1/05, A61B 5/00

(54) **IMPLANTIERBARE ELEKTRODENANORDNUNG**
IMPLANTABLE ELECTRODE ARRANGEMENT
ENSEMBLE ÉLECTRODE IMPLANTABLE

(30) Priorität: 07.10.2014 DE 102014014927
(43) Veröffentlichungstag der Anmeldung: 16.08.2017
(73) Patentinhaber: Neuroloop GmbH, 79108 Freiburg (DE)
(72) Erfinder: PLACHTA, Dennis, 79279 Vörstetten (DE); GIERTHMÜHLEN, Mortimer, 79104 Freiburg (DE); STIEGLITZ, Thomas, 79110 Freiburg (DE); ZENTNER, Josef, 79117 Freiburg (DE)
(74) Vertreter: Rösler, Uwe
(86) Internationale Anmeldenummer: PCT/EP2015/073129
(87) Internationale Veröffentlichungsnummer: WO 2016/055512

(56) Entgegenhaltungen:
- EP-A1- 2 263 745
- WO-A1-93/20887
- WO-A1-2013/150524
- DENNIS T T PLACHTA / OSCAR COTA / THOMAS STIEGLITZ / MORTIMER GIERTHMUEHLEN: "Selektive Ableitung und Stimulation fuer ein blutdrucksenkendes Implantat unter Verwendung von Vielkanal-Cuff-Elektroden", TECHNISCHES MESSEN TM, R.OLDENBOURG VERLAG. MUNCHEN, DE, Bd. 80, Nr. 5, 1. Januar 2013 (2013-01-01) , Seiten 163-172, XP009187692, ISSN: 0171-8096 in der Anmeldung erwähnt

## Beschreibung

### Technisches Gebiet

Die Erfindung bezieht sich auf eine implantierbare Elektrodenanordnung zur ortsselektiven Erfassung neuronaler elektrischer Signale, die sich längs wenigstens einer in einem Nervenfaserbündel enthaltenden Nervenfaser ausbreiten, sowie zur selektiven elektrischen Stimulation der wenigstens einen selektierten Nervenfaser, mit einem biokompatiblen Trägersubstrat, das zumindest einen Trägersubstratbereich aufweist, der um das Nervenfaserbündel manschettenartig anlegbar ist und eine im implantierten Zustand dem Nervenfaserbündel zugewandt orientierte, geradzylinderförmige Trägersubstratoberfläche aufweist, die eine axiale sowie eine in Umfangsrichtung orientierte Erstreckung besitzt und an der eine erste Elektrodenanordnung angebracht ist. Die erste Elektrodenanordnung umfasst in axialer Abfolge jeweils voneinander beabstandet wenigstens drei erste Elektrodenstrukturen mit jeweils wenigstens zwei in Umfangsrichtung verteilt angeordneten ersten Elektrodenflächen, sowie wenigstens zwei axial zueinander beabstandete, sich in Umfangsrichtung erstreckende und jeweils eine Ringform annehmende, erste Elektrodenstreifen, die die wenigstens drei Elektrodenstrukturen axial beidseitig einschließen. Die erste Elektrodenanordnung ist mit einem Signaldetektor und -generator verbindbar, d.h. die Elektrodenanordnung ist über eine trennbare elektrische Schnittstelle, bspw. in Form einer Steckereinheit mit dem Signaldetektor und -generator verbunden, oder direkt, d.h. untrennbar verbunden.

### Stand der Technik

Die arterielle Hypertonie ist eine weltweit verbreitete, typische Zivilisationskrankheit, die das Leben von Millionen Patienten bedroht und gleichzeitig im hohen Maße die Gesundheitssysteme belastet. Bisher bekannte therapeutische Maßnahmen gründen auf der Verabreichung blutdrucksenkender Arzneimittel, wie bspw. ACE-Hemmer, Betablocker etc., diese weisen jedoch neben der erwünschten blutdrucksenkenden Wirkung beachtliche Nebenwirkungen auf, wie bspw. Bradykardie, Herzinsuffizienz, Asthmaanfälle etc. auf. Hinzukommt, dass trotz der Entwicklung neuer blutdrucksenkender Medikamente bei bis zu 30% aller Patienten bei entsprechender Medikation kein adäquater Zielblutdruck erreicht werden kann, siehe Beitrag von H. R. Black, et al., "Principal results of the controlled onset Verapamil investigation of cardiovascular end points (Convince), TRIAL. Jama, 289 (16), S. 2073 - 2082), 2003.

Einen anderen therapeutischen Ansatz zur Begegnung von Bluthochdruck verfolgt eine Studie seitens der Anmelderin, die in dem Artikel von Dennis T. T. Plachta, Oscar Cota, Thomas Stieglitz, Mortimer Gierthmuehlen, "Selektive Ableitung und Stimulation für ein blutdrucksenkendes Implantat unter Verwendung von Vielkanal-Cuff-Elektroden", tm - Technisches Messen, 2013, Vol.80 (5), pp.163-172. publiziert worden ist. Die anhand von an Ratten durchgeführten Tierversuchen gewonnenen Erkenntnisse begründen die Möglichkeit mittels einer an einem Nervenfaserbündelabschnitt des Nervus Vagus implantierten Elektrodenanordnung neuronale elektrische Signale ortsaufgelöst aus dem Nervenfaserbündelabschnitt zu detektieren sowie elektrische Signale an selektierte Nervenfasern zu deren Stimulierung zu Zwecken einer technisch initiierten Blutdruckreduzierung zu applizieren. Eine derartige Vagusnervstimulation besitzt somit grundsätzlich das Potential als Alternative zur Behandlung des therapierefraktären Blutdruckes etabliert zu werden.

Das Konzept der selektiven Vagusnervstimulation stützt sich auf Erfahrungen der seit vielen Jahren angewandten und etablierten neuromodulatorischen Therapie schwerer Formen der Epilepsie, bei der der Vagusnerv mit Hilfe einer implantierten Elektrodenanordnung gesamtheitlich elektrisch stimuliert wird, um bei sich anbahnenden epileptischen Anfällen zumindest deren Ausmaß in Hinblick auf Stärke und zeitliche Dauer abzumildern, siehe hierzu F. Sidiqui, et al., "Cumulative effect of Vagus nerve stimulators on intractable seizures observed over a period of 3 years", Epilepsy and Behavior, 18(3), S. 299 - 302, 2010 sowie T. Stieglitz, "Neuroprothetik und Neuromodulation - Forschungsansätze und klinische Praxis bei Therapie und Rehabilitation", Bundesgesundheitsblatt - Gesundheitsforschung - Gesundheitsschutz, 53(8), S. 783 - 790, 2010.

Im Gegensatz dazu gilt es zur chronischen Behandlung von Hypertonie die blutdruckrelevanten Fasern messtechnisch zunächst zu lokalisieren um sie anschließend in geeigneter Weise selektiv elektrisch zu stimulieren. Um den Vagusnerv durch die implantive Maßnahme der Applikation einer Elektrodenanordnung möglichst zu schonen und das Epineurium des Vagusnervs möglichst nicht zu irritieren, wird in dem zitierten Beitrag von Dennis T. T. Plachta et al. der Einsatz einer sog. Cuff-Elektrode vorgeschlagen, die extraneural am Vagusnerv anbringbar ist. Dies hat den Vorteil einer relativ leichten Positionierung der Cuff-Elektrode längs des Vagusnerves und ermöglicht darüber hinaus einen geringfügig invasiv und daher schonend und schnell durchzuführenden chirurgischen Eingriff am Patienten.

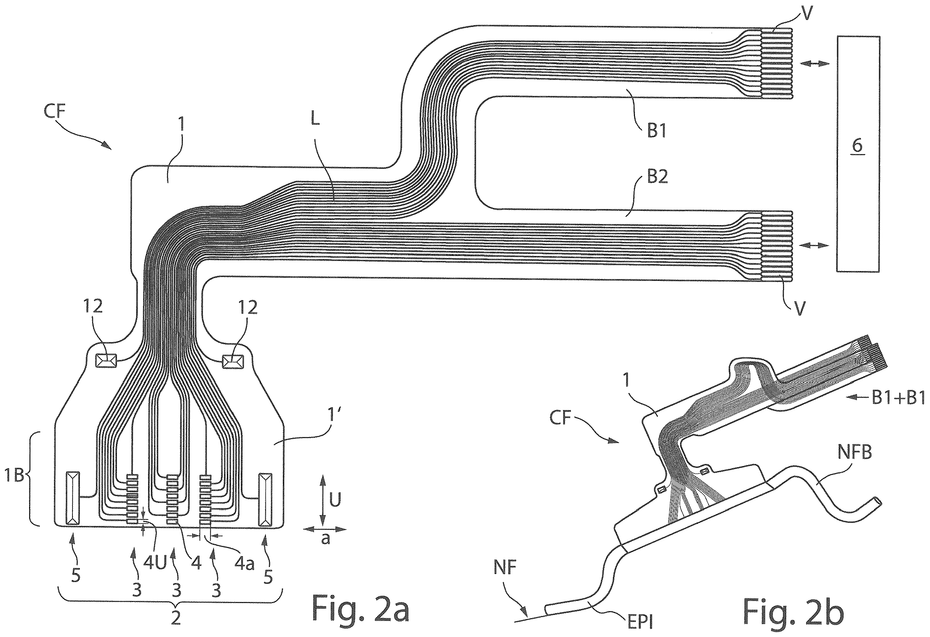

Für die natürliche Blutdruckregelung dient der Baroreflex, der einen homöostatischen, selbstregulierenden Mechanismus darstellt und bei einem erhöhten Blutdruck reflektorisch unterschiedliche Effektoren aktiviert. U.a. wird hierbei die Herzfrequenz abgesenkt, aber auch die arteriellen Gefäße werden geweitet, um damit den Blutdruck zu senken. Im Falle eines niedrigen Blutdruckes wird der Baroreflex unterdrückt, wodurch die Herzfrequenz steigt und Blutgefäße verengt werden damit der Blutdruck wieder steigt. Die sensorischen Eingänge für den Baroreflex stellen sog. Barorezeptoren dar, die sich unter anderem in den Wänden des Aortenbogens befinden. Von dort ziehen die Blutdruckinformationen monosynaptisch längs der blutdruckrelevanten Nervenfasern, im Folgenden als barorezeptive Fasern bezeichnet, in den Hirnstamm. Beim Überschreiten eines Schwellenwertes für den Blutdruck löst der Baroreflex eine Inhibition sympathischer Nervenfasern aus, was zu einer unmittelbaren Absenkung des Blutdruckes führt. Mit Hilfe der unter Bezugnahme auf Figuren 2a, b dargestellten Manschettenelektrode, die in der englischsprachigen Literatur häufig als Cuff-Elektrode bezeichnet wird, ist es möglich, diesen Baroreflexmechanismus zu nutzen, indem die den Hirnstamm zugeführten Druckinformationen selektiv detektiert und gleichfalls selektiv "überschrieben" werden, um den Hirnstamm auf diese Weise eine wesentlich erhöhte Blutdrucksituation zu suggerieren, wodurch eine natürliche signifikante Blutdruckabsenkung initiiert wird.

Figur 2a zeigt die bekannte Manschettenelektrode CE in einer flächigen Draufsicht in einem planar entfalteten Zustand. Figur 2b zeigt die Manschettenelektrode CE im implantierten Zustand, in dem Bereiche B1, B2 der Manschettenelektrode CE zu Zwecken einer raumsparenden Form aufeinander gefaltet sind und darüber hinaus ein mit einer Elektrodenanordnung 2 versehener Trägersubstratbereich 1B der Manschettenelektrode CE manschettenartig einen Bereich eines Nervenfaserbündels NFB umfasst.

Die Manschettenelektrode CE besteht aus einem flexiblen, biokompatiblen Trägersubstrat 1, das in der realisierten Ausführungsform eine ca. 11 µm dicke Polyimid-Folie ist, an deren der Zeichenebene in Figur 2a zugewandten Trägersubstratoberseite zu Zwecken der ortsaufgelösten Erfassung neuronaler elektrischer Signale sowie auch zur selektiven elektrischen Stimulation einzelner im Nervenfaserbündel NFB verlaufender Nervenfasern NF eine aus einer Vielzahl einzelner Elektroden zusammengesetzte Elektrodenanordnung 2 aufgebracht ist. Die einzelnen Elektroden der Elektrodenanordnung 2 gelangen in unmittelbaren Oberflächenkontakt mit dem Epineurium E des Nervenfaserbündels NFB, da sich das Trägersubstrat 1 im Trägersubstratbereich 1B durch entsprechende Einprägung einer mechanischen Folienvorspannung selbständig unter Ausbildung einer dem Nervenfaserbünde NFB zugewandt orientierten geradzylinderförmigen Trägersubstratoberfläche 1' aufrollt, wie dies in Figur 2b ersichtlich ist. So nehmen die einzelnen Elektroden der Elektrodenanordnung 2 eine in Umfangsrichtung U um das Nervenfaserbündel NFB gebogene ringförmige Raumform an.

Sowohl zur ortsselektiven Erfassung neuronaler elektrischer Signale als auch zur selektiven elektrischen Stimulation wenigstens einer Nervenfaser NF dienen drei axial jeweils gleich zueinander beabstandet angeordnete, erste Elektrodenstrukturen 3, die in Umfangsrichtung U wenigstens zwei, im illustrierten Ausführungsbeispiel gemäß Figur 2a, b jeweils acht erste Elektrodenflächen 4 umfassen. Die jeweils acht einer ersten Elektrodenstruktur 3 zugehörigen ersten Elektrodenflächen 4 sind in Umfangsrichtung U gleich verteilt, d. h. in 45° Winkelabständen, angeordnet. Dies ermöglicht eine achtfache, in Umfangsrichtung unterteilte Ortsselektivität zur ortsselektiven Erfassung neuronaler elektrischer Signale aus dem zu untersuchenden Nervenfaserbündel NFB. Die jeweils axial beidseitig neben den drei ersten Elektrodenstrukturen 3 angeordneten ersten Elektrodenstreifen 5, die das Nervenfaserbündel NFB vollständig ringförmig umfassen, dienen im Falle der ortsselektiven Erfassung neuronaler elektrischer Signale als Massepotential; gilt es hingegen selektiv ausgewählte Nervenfasern NF innerhalb des Nervenfaserbündels NFB elektrisch zu stimulieren, so dienen diese ersten Elektrodenstreifen 5 jeweils als Anode bzw. als Gegenpolarität.

Die Dreifach- bzw. Tripol-Anordnung der jeweils ersten Elektrodenstrukturen 3, über deren jeweils erste Elektrodenflächen 4 monopolar neuronale elektrische Signale erfasst bzw. elektrische Signale zu Zwecken der ortsselektiven Stimulation abgegeben werden können, ermöglicht es Impedanzänderungen aufgrund von Gewebewachstum an den metallischen Elektrodenflächen 4 festzustellen und auswertetechnisch zu eliminieren, zum anderen können blutdruckrelevante neuronale Signale, die mit einem leichten zeitlichen Versatz durch die Tripolanordnung axial längs einer entsprechenden Nervenfaser NF laufen, mittels geeigneter tripolarer Verstärkung detektiert werden. Neben den vorstehend bezeichneten ersten Elektrodenstrukturen 3 sowie ersten, jeweils eine Ringform annehmenden Elektrodenstreifen 5, die allesamt an der in Figur 2a der Zeichenebene zugewandten Trägersubstratoberfläche 1' aufgebracht sind und über entsprechend elektrische Leitbahnen L proximalseitig an Verbindungsstrukturen V enden, befinden sich rückseitig am Trägersubstrat 1 Referenzelektroden 12, die zum einen zur Erfassung des intrakorporalen elektrischen Hintergrundmassensignals bzw. Rauschniveaus dienen, das der Signalauswertung zugrunde gelegt wird, zum anderen die Möglichkeit eröffnen, EKG-Signale mit Hilfe der Manschettenelektrode CE zu erfassen. Die als Manschettenelektrode CE implantierbare Elektrodenanordnung ist über die elektrischen Verbindungsstrukturen V mit einem hermetisch gekapselten Signaldetektor und -generator 6 verbindbar, der ebenfalls als Implantat ausgebildet ist.

Mit der bekannten implantierbaren Elektrodenanordnung konnte im Rahmen von Tierversuchen an Ratten gezeigt werden, dass mit Hilfe der gleichverteilt um das Nervenfaserbündel NFB tripolar angeordneten, insgesamt 24 ersten Elektrodenflächen Blutdruck korrelierte, neuronale elektrische Zeitsignale, im Folgenden als barorezeptive Signale bezeichnet, erfasst werden können, die darüber hinaus hinsichtlich ihrer in Umfangsrichtung abhängigen Signalpegel zur Ortung der barorezeptiven Nervenfasern dienen. Die Stimulation erfolgte tripolar jeweils mit jener Elektrodenfläche 4 oder jenen Elektrodenflächen 4 der mittig angeordneten ersten Elektrodenstruktur 3 der Tripol-Anordnung, über die bei der Detektion der jeweils größte Signalpegel unter den barorezeptiven Signalen erfasst wurde. Es konnte gezeigt werden, dass durch selektive Stimulation barorezeptiver Nervenfasern der Blutdruck zuverlässig deutlich gesenkt werden kann, wobei sich lediglich eine sehr schwache Bradykardie (Pulsreduzierung unter 60 Schläge pro Minute) sowie eine kaum nennenswerte Bradypnoe (Reduzierung der Atmung unter 20 Atemzüge pro Minute) einstellten.

Zur selektiven elektrischen Stimulation der barorezeptiven Nervenfasern wurden elektrische Stimulationssignale mit einer Stimulationsfrequenz jeweils zwischen 30 bis 50 Hz, einer Stimulationsdauer von 0,1 bis 0,5 msec sowie einer Stimulationsamplitude von 0,4 bis 1,5 mA an die jeweils selektierten Elektrodenflächen 4 der mittig angeordneten Elektrodenstruktur appliziert. Hierbei erfolgte die elektrische Stimulation längs der barorezeptiven Nervenfasern isotrop, d. h. ohne Vorgabe einer festgelegten Signalausbreitungsrichtung, so dass sich die elektrischen Stimulationssignale sowohl längs afferenter sowie auch efferenter Nervenfasern ausbreiten konnten. Letztere können einen direkten, unkontrollierten Einfluss auf die Herzaktivität ausüben, was zu unerwünschten Nebenwirkungen führen kann, insbesondere bei im Vergleich zu Ratten größeren Lebewesen.

### Darstellung der Erfindung

Der Erfindung liegt die Aufgabe zugrunde eine implantierbare Elektrodenanordnung der vorstehend bezeichneten Art zur ortsselektiven Erfassung neuronaler elektrischer Signale, die sich längs wenigstens einer in einem Nervenfaserbündel enthaltenen Nervenfasern ausweiten, sowie zur selektiven elektrischen Stimulation wenigstens einer Nervenfaser mit den Merkmalen des Oberbegriffes des Anspruches 1 derart weiterzubilden, so dass Vorkehrungen getroffen werden sollen, mögliche Nebenwirkungen bedingt durch unkontrollierte Signalausbreitungseffekte der längs in barorezeptiven Nervenfasern selektiv eingekoppelten, elektrischen Stimulationssignale möglichst vollständig auszuschließen. Insbesondere gilt es Vorkehrungen zu treffen eine Ausbreitung von elektrischen Stimulationssignalen längs efferenter Nervenfasern zu unterbinden ohne dabei einen signifikant nachhaltigen Einfluss auf nicht barorezeptive, afferente sowie efferente Nervenfasern innerhalb des Nervenfaserbündels auszuüben.

Die Lösung der der Erfindung zugrunde liegenden Aufgabe ist im Anspruch 1 angegeben. Den Lösungsgedanken in vorteilhafter Weiser weiterbildende Merkmale sind Gegenstand der Unteransprüche sowie der weiteren Beschreibung unter Bezugnahme auf die Ausführungsbeispiele zu entnehmen.

Die lösungsgemäße implantierbare Elektrodenanordnung gemäß den Merkmalen des Oberbegriffes Anspruches 1 zeichnet sich dadurch aus, dass in axialer Abfolge auf der dem Nervenfaserbündel zugewandten geradzylinderförmigen Trägersubstratoberfläche axial neben der ersten Elektrodenanordnung wenigstens eine zweite Elektrodenanordnung angeordnet ist, die wenigstens zwei axial voneinander beabstandete, sich in Umfangsrichtung erstreckende und jeweils eine Ringform annehmende zweite Elektrodenstreifen sowie axial zwischen den wenigstens zwei zweiten Elektrodenstreifen wenigstens eine jeweils wenigstens zwei in Umfangsrichtung gleich verteilt angeordnete zweite Elektrodenflächen umfassende zweite Elektrodenstruktur umfasst, wobei die zweite Elektrodenanordnung zumindest mit dem Signalgenerator oder einem weiteren Signalgenerator verbunden ist.

Die eingangs erläuterte als Manschettenelektrode ausgebildete, implantierbare Elektrodenanordnung, wie sie unter Bezugnahme auf die Figuren 2a und b erläutert worden ist, ist lösungsgemäß durch wenigstens eine zur Inhibition einer unidirektionalen elektrischen Signalübertragung längs wenigstens einer selektierten Nervenfaser innerhalb eines Nervenfaserbündels ausgebildeten zweiten Elektrodenanordnung ergänzt worden.

Die ebenso auf dem gleichen, einstückig zusammenhängend ausgebildeten Trägersubstrat an der gleichen Trägersubstratoberfläche, wie die erste Elektrodenanordnung aufgebrachte zweite Elektrodenanordnung steht in einer räumlich festen Zuordnung zur ersten Elektrodenanordnung, insbesondere zu den ersten Elektrodenflächen der wenigstens drei ersten Elektrodenstrukturen, mit deren Hilfe barorezeptive Nervenfasern innerhalb des Nervenfaserbündels ortsselektiv erfasst und darüber hinaus selektiv elektrisch stimulierbar sind. In Kenntnis der lokalisierten barorezeptiven Nervenfasern lässt sich die zweite Elektrodenanordnung zu Zwecken einer selektiven Inhibition der barorezeptiven Nervenfasern zur Unterdrückung einer Weiterleitung elektrischer Stimulationssignale längs efferenter, d.h. zum Herzen führender Nervenfasern nutzen. Hierzu dienen wenigstens zwei, vorzugsweise vier oder mehr zweite Elektrodenflächen wenigstens einer zweiten Elektrodenstruktur, die gleichfalls wie die ersten Elektrodenflächen einer der wenigstens drei ersten Elektrodenstrukturen in Umfangsrichtung der dem Nervenfaserbündel zugewandt orientierten, sich geradzylinderförmig ausbildenden Trägersubstratoberfläche gleich verteilt angeordnet sind. Zu Zwecken der Inhibition lokalisierter efferenter barorezeptiver Nervenfasern wird wenigstens eine der zweiten Elektrodenflächen der zweiten Elektrodenstruktur elektrisch aktiviert, wodurch es zu einer gezielt, zeitlich begrenzten, selektiven Inhibition der betreffenden efferenten Nervenfaser kommt. Hierbei tritt von der jeweils wenigstens einen aktivierten, zweiten Elektrodenfläche ein elektrisches Polarisationsfeld in das Nervenfaserbündel ein und wechselwirkt vornehmlich mit der zu inhibieren Nervenfaser. Um das sich während der Inhibition in das Nervenfaserbündel ausbreitende elektrische Polarisationsfeld axial zu begrenzen, dienen jeweils zur zweiten Elektrodenstruktur axial beidseitig angebrachte zweite Elektrodenstreifen, die im implantierten Zustand der Manschettenelektrode das Nervenfaserbündel vollständig umschließende Ringelektroden darstellen.

Zu Zwecken der Inhibition ausgewählt efferenter Nervenfasern ist die lösungsgemäß ausgebildete, implantierbare Elektrodenanordnung derart an das Nervenfaserbündel zu applizieren, so dass die lösungsgemäß vorgesehene zweite Elektrodenanordnung dem Herzen, bzw. den barorezeptiven Rezeptoren zugewandt, d.h. caudal, und die erste Elektrodenanordnung, mit der die selektive Erfassung neuronaler elektrischer Signale sowie auch die elektrische Stimulation lokalisierter Nervenfasern vorgenommen wird, dem Gehirn, d.h. rostral, längs des Nervenfaserbündels zugewandt orientiert sind.

Mit Hilfe der zweiten Elektrodenanordnung lässt sich die Inhibition entweder im Wege eines sog. anodalen Blockes oder durch Applikation von sinusförmigen Signalen von Frequenzen im Kilohertzbereich realisieren. Im Falle des anodalen Blocks wird wenigstens eine der zweiten Elektrodenflächen anodisch polarisiert, wodurch ein am Ort der efferenten Nervenfaser vorherrschendes Spannungspotential erzeugt wird, durch das eine aktivierende Stimulation der entsprechenden Nervenfaser unterdrückt wird. Ebenso kann eine Inhibition im Wege einer Hochfrequenzsignalapplikation erzielt werden, bei der an wenigstens einer ausgewählten zweiten Elektrodenfläche ein hochfrequentes elektrisches Inhibitionssignal angelegt wird, wodurch die elektrischen Signalübertragungsmechanismen längs der efferenten Nervenfasern kurzzeitig zum Erliegen kommen.

In beiden Fällen wirkt die lösungsgemäß vorgesehene zweite Elektrodenanordnung aufgrund ihrer räumlich axialen Begrenztheit, die durch den axialen Abstand beider zweiten Elektrodenstreifen gegeben ist, trotz ihrer räumlichen Nähe zur ersten Elektrodenstruktur, immerhin sollte die implantierbare Elektrodenanordnung eine axiale Länge von 4 cm nicht überschreiten, axial räumlich begrenzt längs der zu inhibierenden efferenten Nervenfasern, so dass die gehirnseitig längs des Nervenfaserbündels angeordnete erste Elektrodenanordnung in den jeweils lokalisierten afferenten Nervenfasern zum Gehirn führende elektrische Stimulationssignale unbeeinflusst von dem Inhibitionsmechanismus einkoppeln kann. Auf diese Weise können jegliche Nebenwirkungen, bedingt durch mögliche direkte Stimulationen in Richtung der zum Herz führenden, d.h. efferenten Nervenfasern ausgeschlossen werden.

In vorteilhafter Weise sind die zweiten Elektrodenflächen der zweiten Elektrodenstruktur im implantierten Zustand der Manschettenelektrode längs einer virtuellen Kreislinie gleich verteilt angeordnet, um auf diese Weise relativ zum Umfangsrand eines Nervenfaserbündels lokalisierte efferente Nervenfasern selektiv und effektiv zu inhibieren.

Nicht notwendigerweise jedoch in vorteilhafter Form sind die zweiten Elektrodenflächen in Form und Größe identisch untereinander ausgebildet, wobei ihre axialen Erstreckungen jeweils identisch gewählt sind, gleichsam den axialen Erstreckungen der ersten Elektrodenflächen der ersten drei Elektrodenstrukturen. Die in Umfangsrichtung orientierte Erstreckung der jeweils zweiten Elektrodenflächen ist größer gewählt als die in Umfangsrichtung orientierte Erstreckung der ersten Elektrodenflächen. Somit weisen die zweiten Elektrodenflächen gegenüber den ersten Elektrodenflächen vorzugsweise ein größeres Flächenmaß auf, wodurch die Ortsselektivität, mit der die zweiten Elektrodenflächen bestimmte efferente Nervenfasern elektrisch zu polarisieren vermögen geringer ist als die Ortsselektivität, mit der die ersten Elektrodenflächen lokalisierte Nervenfasern elektrisch zu stimulieren vermögen. Alternativ können die zweiten Elektrodenflächen anstelle einer Rechtecksform auch als Kreisflächen ausgebildet sein. Dies hat den Vorteil, dass sich keine lokalen, durch Kanten oder Ecken bedingten elektrische Potenzialfeldspitzen ausbilden.

Die zweite Elektrodenanordnung ist vorzugsweise in Form einer Tripolar-Elektrodenanordnung ausgebildet, d. h. die zweite Elektrodenstruktur ist axial beidseitig von jeweils einem ringförmig ausgebildeten zweiten Elektrodenstreifen begrenzt, wobei der axiale Abstand zwischen beiden zweiten Elektrodenstreifen längs des Trägersubstrates vorzugsweise zwischen 0,5 cm und 3 cm, insbesondere zwischen 0,75 cm und 1,25 cm gewählt ist. Die ringförmig ausgebildeten zweiten Elektrodenstreifen besitzen vorzugsweise eine axiale Erstreckung zwischen 1µm und 5 mm, vorzugsweise zwischen 100 µm und 4000 µm.

Die zweiten Elektrodenflächen der zweiten Elektrodenstruktur sind axial mittig zwischen beiden zweiten Elektrodenstreifen angeordnet und verfügen über eine axiale Erstreckung, so dass der jeweils axiale Abstand zu den zweiten Elektrodenstreifen größer ist als ihre eigene axiale Erstreckung.

Insbesondere im Hinblick auf die Möglichkeit der Durchführung von depolarisierenden Maßnahmen ist es denkbar, anstelle einer zweiten Elektrodenstruktur drei axial beabstandete zweite Elektrodenstrukturen zwischen den zweiten Elektrodenstreifen anzuordnen, gleichsam zur Ausbildung der jeweils ersten Elektrodenstruktur innerhalb der ersten Elektrodenanordnung. Nur der Vollständigkeit halber sei erwähnt, dass es ebenso denkbar wäre, auch mehr als drei erste und zweite Elektrodenstrukturen zwischen den jeweiligen ersten und zweiten Elektrodenstreifen anzuordnen. So könnten drei, fünf, sieben oder mehr ungeradzahlige erste und/oder zweite Elektrodenstrukturen vorgesehen werden.

In einem nachstehend illustrierten bevorzugten Ausführungsbeispiel umfasst eine zweite Elektrodenstruktur vier zweite Elektrodenflächen, deren Elektrodenflächenmaß jeweils kleiner als ein Viertel der Flächengröße jeweils eines zweiten Elektrodenstreifens gewählt ist. Da die sowohl in der ersten als auch in der zweiten Elektrodenanordnung vorgesehenen ersten bzw. zweiten Elektrodenstreifen jeweils als Masse- oder Gegenpole zur Polarisierung der jeweils ersten bzw. zweiten Elektrodenstruktur dienen, sind aus Gründen ladungssymmetrischer Verhältnisse die Flächengrößen der ersten und zweiten Elektrodenstreifen jeweils identisch gewählt. Vorstellbar ist jedoch auch eine individuell unabhängige Flächengrößenwahl in der Ausbildung der ersten und zweiten Elektrodenstreifen.

Ferner hat es sich als vorteilhaft erwiesen, sämtliche Elektroden der zweiten Elektrodenanordnung, d.h. die zweiten Elektrodenflächen und zweiten Elektrodenstreifen, aus einem elektrisch leitenden Material zu fertigen, das über eine geringere Ladungsübertragungskapazität verfügt, als das Elektrodenmaterial, aus dem die ersten Elektrodenflächen der ersten Elektrodenanordnung bestehen. Als besonders geeignetes Material mit einer besonders hohen Ladungsübertragungskapazität wird Iridium-Oxid zur Ausbildung der jeweils ersten Elektrodenflächen der ersten Elektrodenanordnung verwendet, wohingegen das Material der zweiten Elektrodenflächen und zweiten Elektrodenstreifen aus Platin oder aus einem elektrisch leitfähigen Polymer besteht.

Sämtliche Elektrodenflächen sowohl der ersten als auch zweiten Elektrodenanordnung sind vorzugsweise bündig zur Trägersubstratoberfläche des Trägersubstrats ausgebildet oder gegenüber dieser abgesetzt angeordnet, so dass sie die Trägersubstratoberfläche nicht überragen, um einen möglichst schonenden Oberflächenkontakt zum Epineurium des Nervenfaserbündels herzustellen. Durch den nichtinvasiven Oberflächenkontakt kann die implantierbare Elektrodenanordnung längs des Nervenfaserbündels operativ leicht appliziert und positioniert werden, wobei das Epineurium nur minimal bis nicht irritiert wird.

Um ferner implantationsbedingten Gewebeirritationen entzündlichen Reaktionen entgegen zu treten, bietet es sich an, das aus einem biokompatiblen Polymer bestehende Trägersubstrat zumindest in jenen Bereichen, die mit dem Nervenfaserbündel in unmittelbaren Oberflächenkontakt treten, mit einem Inflammationsreaktionen inhibierenden Wirkstoff zu versehen.
Eine weitere Maßnahme zur Reduzierung mechanischer Reizungen des Nervenfaserbündels, die durch den Oberflächenkontakt mit der manschettenartigen Manschettenelektrode entstehen können, betrifft eine Abrundung axialer Begrenzungskanten der das Nervenfaserbündel umschließenden Trägersubstrats derart, dass das biokompatible Trägersubstrat im Bereich der dem Nervenfaserbündel zugewandt orientierten, geradzylinderförmigen Trägersubstratoberfläche jeweils axial sich gegenüberliegende Randbereiche aufweist, an denen das Trägersubstrat über eine größere Substratdicke verfügt als im übrigen Trägersubstratbereich, wobei die Randbereiche über abgerundete Randkanten verfügen.

Im Bereich der zweiten Elektrodenanordnung, die zur elektrischen Inhibition lokalisierter Nervenfasern dient, sieht eine weitere bevorzugte Ausführungsform wenigstens eine, vorzugsweise mehrere Lichtwellenleiteröffnungen bzw. -aperturen vor, über die Licht durch das Epineurium des Nervenfaserbündel appliziert bzw. eingekoppelt werden kann. Die Lichtwellenleiteröffnungen sind vorzugsweise axial zu beiden zweiten Elektrodenstreifen benachbart angeordnet und in Form, Größe und Verteilung entsprechend den zweiten Elektrodenflächen der zweiten Elektrodenstruktur nachgebildet. Durch Vorsehen mehrerer, räumlich separierter Lichtwellenleiter, die an der Trägersubstratoberfläche dem Nervenfaserbündel zugewandt münden, können dem Nervenfaserbündel einheitliche oder unterschiedliche optische Signale mit unterschiedlichen Wellenlängen zu Zwecken einer optischen Aktivierung neuronaler optogenetischer Reaktionen innerhalb des Nervenfaserbündels appliziert werden. So lassen sich durch eine Vielzahl geeignet angeordneter Lichtwellenaustrittsöffnungen bzw. -aperturen innerhalb des Nervenfaserbündels neuronale Aktivierungs- oder Inhibitionsreaktionen ortsselektiv auslösen, die alternativ oder in Ergänzung zu den über die Elektrodenflächen hervorgerufenen neuronalen Prozessen vorgenommen werden können.

Wie bereits erwähnt, gilt es die lösungsgemäß ausgebildete implantierbare Elektrodenanordnung derart längs des Nervenfaserbündels zu applizieren, so dass die zweite Elektrodenanordnung längs des Nervenfaserbündels in Richtung zum Herzen zugewandt zu liegen kommt. Auf diese Weise ist sichergestellt, dass efferente Nervenfasern inhibiert werden können, wohingegen die dem Gehirn längs des Nervenfaserbündels zugewandt orientierte erste Elektrodenanordnung zu Zwecken der selektiven Stimulation lokalisierter afferenter, d. h. zum Hirn führenden Nervenfasern eingesetzt werden kann. Sollte es einen Bedarf geben, afferente Nervenfasern selektiv zu inhibieren, so kann die lösungsgemäß ausgebildete implantierbare Elektrodenanordnung mit umgekehrter Orientierung längs des Nervenfaserbündels implantiert werden. Eine weitere mögliche Ausführungsform sieht eine zweite inhibierende zweite Elektrodenanordnung vor, die axial neben der ersten Elektrodenanordnung, der zweiten Elektrodenanordnung gegenüberliegend angebracht ist.

Zur Ansteuerung und elektrischen Signal- und Energieversorgung sämtlicher auf dem Trägersubstrat aufgebrachten Elektrodenflächen und Elektrodenstreifen ist wenigstens ein Signaldetektor und -generator vorgesehen, der gemeinsam mit einer elektrischen Energieversorgungseinheit separat zum Trägersubstrat innerhalb einer kapselartigen Einhausung hermetisch eingefasst ist oder als integraler Bestandteil des Trägersubstrates vorgesehen ist. Im Falle einer separaten Ausbildung des Signaldetektors und -generators ist dieser über eine entsprechende elektrische und gegebenenfalls optische Schnittstelle mit der lösungsgemäß ausgebildeten implantierbaren Elektrodenanordnung verbindbar.

Die intrakorporale Implantation der das Nervenfaserbündel manschettenartig umgebenden Elektrodenanordnung ist zudem mit dem grundsätzlichen Problem konfrontiert, dass die auf dem Polyimid-Trägersubstrat aufgebrachten Elektrodenstreifen und Elektrodenflächen einem andauernden feuchten Milieu ausgesetzt sind, wodurch Degradationserscheinungen insbesondere an den flächigen Verbindungen zwischen den Elektrodenflächen und dem Polyimid-Trägersubstrat auftreten können, die zu lokalen Ablösungen und damit verbunden zumindest zu Kontaktdegradierungen führen, durch die letztlich die elektrische Effizienz der Elektrodenanordnung beeinträchtigt wird. Um diesen Milieu bedingten Ablösungserscheinungen zwischen metallischen Elektrodenflächen und dem Polyimid-Trägersubstrat zu begegnen, verfügen in einer bevorzugten Ausführungsform zumindest die ersten und zweiten Elektrodenstreifen jeweils über wenigstens eine lokale Öffnung, wobei die ersten und zweiten Elektrodenstreifen derart mit dem Trägersubstrat bzw. der Trägersubstratoberfläche flächig verbunden sind, so dass das Polymer bzw. Polyimid, aus dem das Trägersubstrat besteht, die wenigstens eine Öffnung wenigstens teilweise durchdringt. Hierdurch ist eine verbesserte mechanische Verankerung der jeweiligen Elektrodenstreifen mit dem Trägersubstrat geschaffen.

Eine weitere Möglichkeit für eine dauerstabile Verbindung zwischen den Elektrodenflächen bzw. Elektrodenstreifen und dem biokompatiblen Polyimid bzw. Polymermaterial des Trägersubstrats spiegelt sich in einer speziellen Ausbildungsform der Elektrodenflächen bzw. Elektrodenstreifen wider, sowie einer dadurch möglichen speziellen Integration der Elektroden in das Trägersubstrat. Hierzu weisen insbesondere die ersten und zweiten Elektrodenstreifen jeweils eine metallische Basisplatte mit einer ebenen Ober- und Unterseite auf, mit wenigstens einem, vorzugsweise eine Vielzahl die Oberseite der Basisplatte orthogonal lokal überragenden Strukturelementen, die vorzugsweise säulen-, rippen-, hülsen oder stegartig ausgebildet sind. Die metallische Basisplatte ist vollständig von dem biokompatiblen Polymer des Trägersubstrats umschlossen, mit Ausnahme eines ersten Oberflächenbereiches des wenigstens einen Strukturelementes, der der Trägersubstratoberfläche zugewandt orientiert ist und diese nicht überragt. Somit reduziert sich die frei an der Trägersubstratoberfläche zugängliche Elektrodenkontaktfläche, jedoch ist aufgrund der hermetischen Umschließung der Basisplatte sowie der einstückig daran verbundenen Strukturelemente mit Ausnahme der der Trägersubstratoberfläche zugewandt orientierten Oberflächenbereiche vollständig vom biokompatiblen Polymer des Trägersubstrates umschlossen. Ein Eindringen von milieubedingter Flüssigkeit bzw. Feuchtigkeit zwischen den Elektrodenstreifen und dem biokompatiblen Polymer des Trägersubstrates wird erheblich erschwert, so dass Degradationserscheinungen weitgehend ausgeschlossen werden können. In einer weiteren bevorzugten Ausführungsform ist vorzugsweise zwischen der Unterseite der metallischen Basisplatte und dem biokompatiblen Polymer des Trägersubstrates eine Haftvermittlerschicht oder eine Haftvermittlerschichtanordnung eingebracht, die mögliche feuchtigkeitsbedingten Ablöseerscheinungen entgegen tritt.

Auf weitere bevorzugte Ausführungsformen in Bezug auf die mögliche Ausgestaltung der Elektrodenstreifen wird in Verbindung mit den nachfolgenden Figuren erläutert.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1: Draufsicht einer schematisierten implantierbaren Elektrodenanordnung mit einer zweiten Elektrodenanordnung zu Inhibition selektiver Nervenfasern,
- Fig. 2a, b: Darstellungen einer an sich bekannten implantierbaren Elektrodenanordnung zur ortsselektiven Erfassung neuronaler elektrischer Signale sowie selektiver elektrischer Stimulation einzelner Nervenfasern,
- Fig. 3a: Darstellung eines Elektrodenstreifens mit Öffnung,
- Fig. 3b: detaillierte Darstellung eines in das Trägersubstrat integrierten Elektrodenstreifens,
- Fig. 3c: Alternative Ausbildung eines Strukturelementes,
- Fig. 4a-f: Darstellungen einer die implantierbare Elektrodenanordnung zusätzlich verstärkende Manschette, und
- Fig. 5: Hydraulische Applikationsstrukturen der implantierbaren Elektrodenanordnung.

### Wege zur Ausführung der Erfindung, gewerbliche Verwendbarkeit

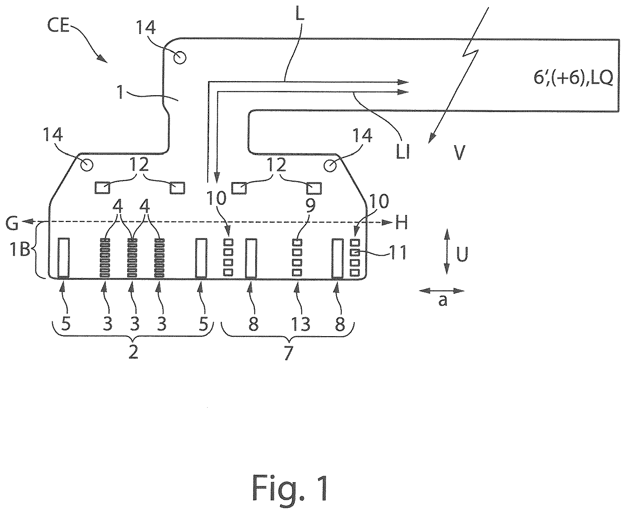

Figur 1 zeigt eine schematische Draufsicht einer lösungsgemäß ausgebildeten, implantierbaren Manschettenelektrode CE, auf deren vorzugsweise aus Polyimid bestehenden Trägersubstrat 1, neben der zur ortsselektiven Detektion neuronaler elektrischer Signale sowie zur selektiven elektrischen Stimulation einzelner Nervenfasern vorgesehenen ersten Elektrodenanordnung 2 eine zur Inhibition wenigstens einer selektierten Nervenfaser dienende zweite Elektrodenanordnung 7 aufgebracht ist. Zur Vermeidung von Wiederholungen wird auf die Erläuterung der einzelnen Elektroden der ersten Elektrodenanordnung 2 auf die vorstehende Beschreibung der Figuren 2a und b verwiesen.
Die zur Signalausbreitung längs efferenter, hier zum Herzen H, führende Nervenfasern inhibierende zweite Elektrodenanordnung 7 umfasst zwei axial beabstandete zweite Elektrodenstreifen 8, zwischen denen mittig eine zweite Elektrodenstruktur 13 vorgesehen ist, die aus vier separat zueinander angeordneten zweiten Elektrodenflächen 9 besteht. Sämtliche Elektroden 8, 13 der zweiten Elektrodenanordnung 2 sind über auf dem Trägersubstrat 1 aufgebrachte oder in dieses intergierte elektrische Leiterbahnen L mit einem Signalgenerator 6' verbunden bzw. verbindbar, der gemeinsam mit dem Signaldetektor und -generator 6 sowie mit einer Energiequelle in einer separat gekapselten, implantierbaren Einheit integriert ist. Die elektrische Leiterbahnen L können optional eine trennbare Verbindungsstruktur V aufweisen.

Optional umfasst die zweite Elektrodenanordnung 2 optische Lichtwellenleiteranordnungen 10, die jeweils vier in Umfangsrichtung U verteilt angeordnete, separate Lichtwellenleiteröffnungen 11 umfassen. Die Lichtwellenleiter LI zu den einzelnen Lichtwellenleiteröffnungen bzw. -aperturen 11 verlaufen innerhalb des Trägersubstrats 1 und können proximalseitig mit einer einheitlichen Lichtquelle LQ oder mit separaten Lichtquellen LQ unterschiedlicher Lichtwellenlängen kombiniert werden, um optogenetisch selektiv aktivierte Stimulierungen und/oder optisch aktivierte und selektive Inhibitionen längs bestimmter Nervenfasern hervorzurufen.

Die geometrische Form- und Größenwahl der einzelnen Elektroden, d. h. der ersten und zweiten Elektrodenstreifen 5, 8 sowie der ersten und zweiten Elektrodenflächen 4, 9 können grundsätzlich individuell aufeinander abgestimmt vorgenommen werden und richten sich insbesondere nach dem Durchmesser des Nervenfaserbündels, um das die implantierbare Manschettenelektrode CF anlegbar ist. So entspricht die in Umfangsrichtung U orientierte Erstreckung der ersten und zweiten Elektrodenstrukturen und Elektrodenstreifen sowie gegebenenfalls der optischen Lichtwellenleiteranordnungen 10 vorzugsweise dem Umfangsrand des mit der Manschettenelektrode CE zu umwickelnden Nervenfaserbündels. Der axiale Abstand der tripolaren Elektrodenanordnung sollte vorzugweise auf den Durchmesser und den damit resultierenden Abstand der sogenannten Ranvierschen Schnürringe bei myelinisierten Nervenfasern der zu erregende Nervenfasern angepasst werden. In dem in Figur 1 dargestellten Ausführungsbeispiel sind die Elektroden als rechteckförmige Elektrodenflächen dargestellt. In vorteilhafter Weise, so insbesondere zu Zwecken der Vermeidung von an Elektroden-Rechteckkanten auftretenden Feldlinienverdichtungen, bietet es sich an die Elektrodenflächen zumindest mit abgerundeten Ecken auszubilden.

So gilt es beim Menschen bestimmte, große und myelinisierte Fasern zu inhibitieren oder zu aktivieren. Dies ist nur an den Stellen längs der Nervenfaser möglich, an denen diese Fasern nicht myelinisiert sind, d.h. an den sogenannten Ranvierschen Ringen. Mit zunehmendem Durchmesser der Nervenfaser sind die Intervalle, d.h. die axialen Abstände zwischen den Ranvierschen Ringen größer, dementsprechend gilt es den axialen Abstand zwischen beiden axial beabstandeten ersten Elektrodenstreifen 5 in etwa gleich lang wie der axiale Abstand der Ringe oder etwas größer zu wählen, um mit hinreichend großer statistischer Wahrscheinlichkeit auch die Ranvierschen Ringe sehr großer Fasern zu erreichen. Entsprechendes gilt vorzugsweise auch für die axiale Beabstandung der zweiten Elektrodenstreifen 8.

Die axiale Gesamterstreckung der gesamten Manschettenelektrode CE sollte an die intrakorporalen Größenverhältnisse des jeweiligen Nervenfaserbündels angepasst sein und typischer Weise 4 cm nicht überschreiten.

Die rückseitig an dem Trägersubstrat 1 angebrachten zusätzlichen Referenzelektrodenflächen 12 dienen der Erfassung des intrakorporal erfassbaren Rauschpegels somit bedarfsweise von EKG-Signalen.

Zusätzlich verfügt das Trägersubstrat 1 über wenigstens einen, vorzugsweise zwei oder drei durch Metallringstrukturen verstärkte Öffnungen 14, die zur Befestigung der implantierten Elektrodenanordnung CF am Nervenfaserbündel dienen. Die Befestigung erfolgt mit Hilfe eines chirurgischen Fadens, der jeweils wenigstens einmal durch die Öffnungen 14 gefädelt und in dem das Nervenfaserbündel umgebenden Gewebe vernäht wird. Im Unterschied zu dem zu einem Geradzylinder aufgerollten Bereich 1B des Trägersubstrats, auf dem die ersten und zweiten Elektrodenanordnungen 2 und 7 aufgebracht sind, so dass sie das Epineurium des Nervenfaserbündels im implantierten Zustand oberflächig berühren, steht das sich an den Trägersubstratbereich 1B anschließende Trägersubstrat 1 in Art einer ebenen Fahne seitlich vom Nervenfaserbündel ab und ragt in das umliegende Gewebe hinein. Die Metallringstrukturen 14 sollen helfen die längs des chirurgischen Fadens wirkenden Befestigungskräfte mechanisch sicher aufzunehmen und einschneidende Schädigungen am Trägersubstrat zu verhindern.

Zur manschettenartigen Umwicklung der implantierbaren -Elektrodenanordnung CF um ein nicht weiter dargestelltes Nervenfaserbündel ist die zweite Elektrodenanordnung 7 auf die zum Herzen führenden Seite H längs des Nervenfaserbündels anzuordnen. Die zweite, der selektiven Detektion sowie auch selektiven Stimulation lokalisierter Nervenfasern dienende Elektrodenanordnung 2 ist längs des Nervenfaserbündels gehirnseitig G angebracht.

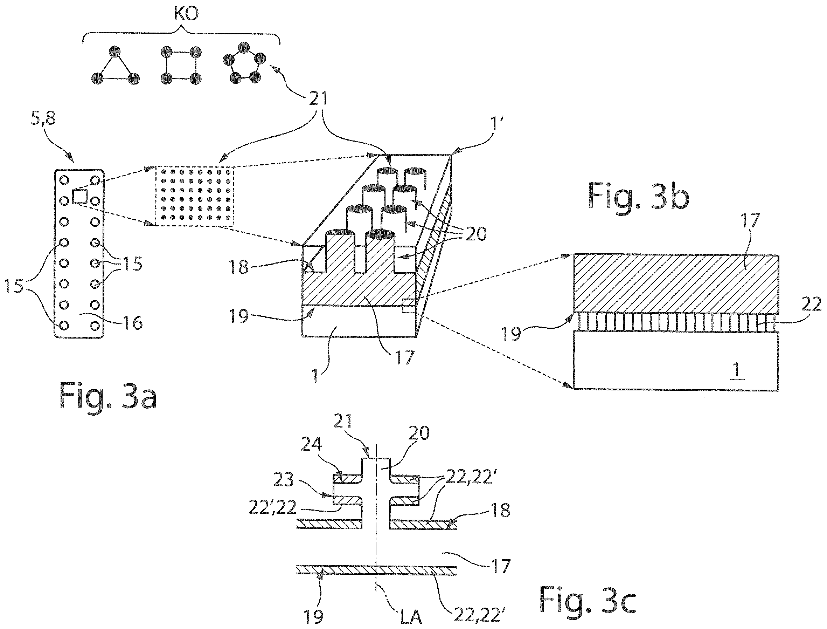

Vorzugsweise sind die ersten und zweiten Elektrodenstreifen 5, 8 sowie die ersten und zweiten Elektrodenflächen 4, 9 auf das Trägersubstrat aufgedampft oder aufgesputtert, eine galvanische Verstärkung ist denkbar. Auch Laserstrukturierung von dünnen Metallfolien ist als Technologie möglich. Für eine dauerhafte Fügung insbesondere der ersten und zweiten Elektrodenstreifen 5, 8 an das Trägersubstrat 1 weisen die Elektrodenstreifen lokale Öffnungen 15 auf, siehe Figur 3a, durch die zumindest teilweise das Polymermaterial des Trägersubstrats 1 hindurchtritt bzw. hindurchragt. Die Elektrodenoberfläche 16 jeweils der ersten und zweiten Elektrodenstreifen 5, 8 sind im Übrigen bündig zur Trägersubstratoberseite 1' angeordnet und kontaktieren die Oberfläche des Nervenfaserbündels unmittelbar.

Um die Fügung der Elektrodenstreifen 5, 8 dauerhaft zu verbessern, wird in einem bevorzugten Ausführungsbeispiel vorgeschlagen, die Elektrodenstreifen in der folgenden Weise weitgehend in das Trägersubstrat zu integrieren, siehe hierzu Figur 3b:
Die Elektrodenstreifen 5, 8 weisen jeweils eine metallische Basisplatte 17 auf, die eine Oberseite 18 und eine Unterseite 19 vorsieht. Einstückig mit der Oberseite 18 der Basisplatte 17 sind über die Oberseite 18, vorzugsweise über die gesamte Oberseite flächig verteilt, orthogonal erhabene Strukturelemente 20 vorgesehen, vorzugsweise in Form säulen-, rippen-, steg- oder hülsenartiger Fortsätze, die über einen der Trägersubstratoberfläche 1' zugewandten Oberflächenbereich 21 verfügen, der in unmittelbarer Anlage mit dem Epineurium des Nervenfaserbündels gelangen kann. Zusätzlich ist in vorteilhafter Weise eine Haftvermittlerschicht 22 zumindest zwischen der Unterseite 19 und dem die Basisplatte 17 umgebenden Polymermaterial des Trägersubstrats 1 vorgesehen. Die Haftvermittlerschicht 22 kann zudem auch auf der Oberseite 18 aufgebracht sein. Besonders geeignete Haftvermittlerschichten bestehen aus Siliziumcarbid (SiC) sowie Diamond like Carbon (DLC). Vorzugsweise werden die Elektrodenstreifen 5, 8 aus Iridiumoxid gefertigt, das zu jenen Materialien mit einer der höchsten Ladungsübertragungskapazitäten zu zählen ist.

Eine weitere verbesserte Variante zur Ausbildung der Strukturelemente 20, die verteilt auf der Oberseite der Basisplatte 17 aufgebracht sind, ist in Figur 3c illustriert. Figur 3c zeigt den Längsschnitt durch ein Strukturelement 20, das eine orthogonal zur Oberseite 18 der metallischen Basisplatte 17 orientierte Längserstreckung LA aufweist, längs der das Strukturelement 20 wenigstens einen zweiten Oberflächenbereich 23 vorsieht, der parallel zur Oberseite 18 der metallischen Basisplatte 17 orientiert ist und auf dem die Haftvermittlerschicht 22 oder eine Haftvermittlerschichtanordnung 22' aufgebracht ist. Der zweite Oberflächenbereich 23 ist vom ersten Oberflächenbereich 18 beabstandet angeordnet und getrennt durch die Haftvermittlerschicht (22) bzw. die Haftvermittlerschichtanordnung (22') vollständig von dem biokompatiblen Polymer umgeben. Der zweite Oberflächenbereich ist, wie aus der Figur 3c zu entnehmen ist, der Oberseite 18 der Basisplatte 17 zugewandt orientiert. Selbstverständlich ist es zusätzlich möglich und vorteilhaft die Haftvermittlerschicht 22 bzw. die Haftvermittlerschichtanordnung 22' sowohl an einem dritten Oberflächenbereich 24, der dem zweiten Oberflächenbereich 23 gegenüberliegt und/oder an der Ober- und/oder Unterseite 18, 19 der Basisplatte 17 vorzusehen.

Die Anzahl sowie auch Anordnung der einzelnen Strukturelemente 20 kann beliebig gewählt werden, vorzugsweise eignen sich jedoch geometrisch geordnete Konstellationen KO, wie bspw. quadratische, pentagonale, hexagonale oder höherwertige Anordnungsmuster, wie dies aus der Figur 3b zu entnehmen ist.

In einer bevorzugten Anordnung der Basisplatte 3 innerhalb des Trägersubstrats 1 befindet sich die Basisplatte 17 mittig innerhalb des Trägersubstrats 1, d.h. die Dicke der sich an die Unterseite 19 der Basisplatte 17 angrenzenden biokompatiblen Polymerschicht sollte in etwa der Dicke der sich an die Oberseite 18 der Basisplatte 17 angrenzenden Polymerschicht entsprechen. Mit einer derartigen Anordnung der Basisplatte 17 ist der experimentell nachweisbare Vorteil verbunden, dass sich die auf die Basisplatte 17 einwirkenden, materialinhärenten Spannungen kompensieren, die sich während eines Temperprozesses ausbilden. Der Temperprozess ist erforderlich, um eine Materialvorspannung in das Trägersubstrat einzuprägen, durch die sich die implantierbar Manschettenelektrode selbstständig um das Nervenfaserbündel zu wickeln vermag.

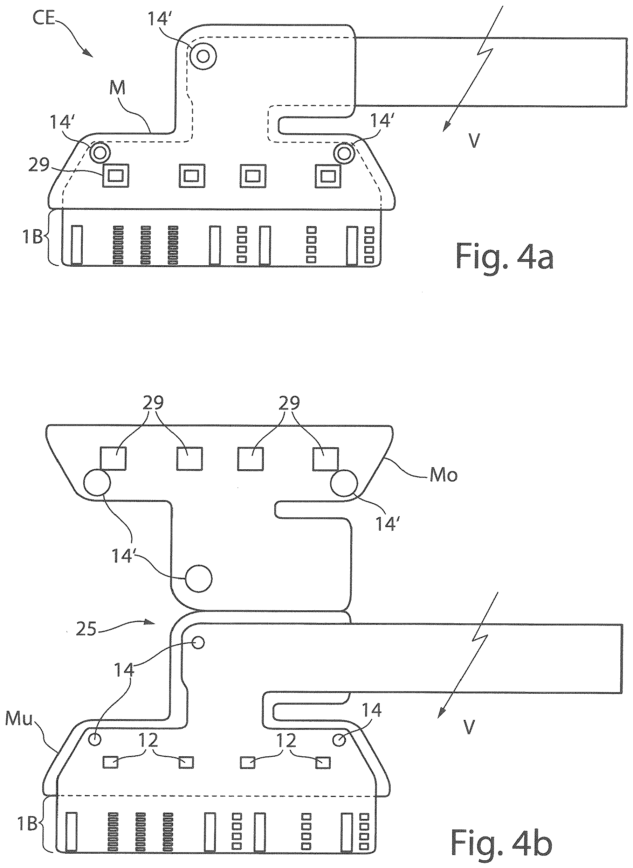

In den Figuren 4a bis f ist eine das Trägersubstrat 1 der implantierbaren Manschettenelektrode CE teilweise umfassende Manschette M dargestellt, die jenen Bereich des Trägersubstrates 1 sowohl an dessen Unter- sowie auch Oberseite umfasst, der sich unmittelbar an den Trägersubstratbereich 1B anschließt und sich im Unterschied zum Trägersubstratbereich 1B nicht im Wege einer materialinhärenten mechanischen Vorspannung selbständig geradzylinderförmig verformt und auf diese Weise bündig an das Epineurium des Nervenfaserbündels im implantierten Zustand in Anlage gebracht wird.

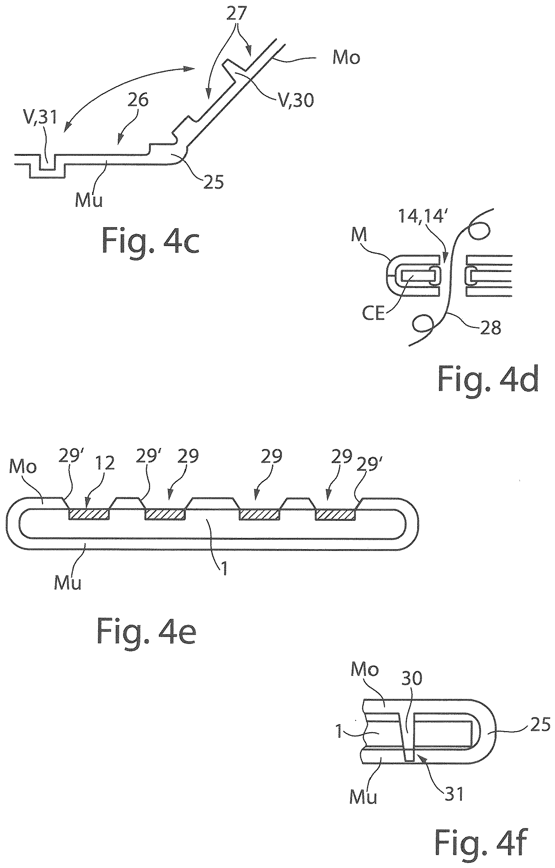

Die Manschette M dient in erster Linie einem verbesserten Handling der implantierbaren Manschettenelektrode CE, die aufgrund ihrer sehr geringen Trägersubstratdicke sowie der auf die Trägersubstratoberfläche aufgebrachten filigranen Elektrodenanordnungen vom Operateur eine besonders sorgfältige Handhabung abverlangt. Die Manschette M ist vorzugsweise einteilig ausgebildet und verfügt über ein Manschettenunterteil Mu sowie ein Manschettenoberteil Mo, die beide über ein Filmscharniergelenk 25 gelenkig verbunden sind, siehe hierzu Figuren 4b und 4c. Das Manschettenunterteil Mu verfügt über eine das Trägersubstrat 1 einbettende Vertiefung 26, in die das Trägersubstrat 1 einsetzbar ist. Im eingesetzten Zustand umfasst das Manschettenunterteil Mu das Trägersubstrat 1 in der aus Figur 4b entnehmbaren, umrahmenden Weise, das heißt das Manschettenunterteil MU ragt seitlich unter dem Trägersubstrat 1 hervor.

Das einstückig mit dem Manschettenunterteil Mu über das Scharniergelenk 25 verbundene Manschettenoberteil Mo ist in Form und Größe an das Manschettenunterteil Mu angepasst und verfügt gleichfalls wie das Manschettenunterteil Mu über eine das Trägersubstrat 1 einbettende Vertiefung 27, so dass im geschlossenem Zustand der Manschette M das Trägersubstrat 1 in der in Figur 4a dargestellten Weise hermetisch umfasst, wobei lediglich der Trägersubstratbereich 1B aus der Manschette M emporragt.

Neben einem verbesserten Handling dient die Manschette M insbesondere auch einer verbesserten Fixierung der Manschettenelektrode CE relativ zum Nervenfaserbündel. Hierzu sehen die Manschettenober- und -unterseiten Mo, Mu jeweils Befestigungsöffnungen 14' vor, siehe Fig. 4a, b, d, die deckungsgleich mit den innerhalb des Trägersubstrates 1 eingebrachten Befestigungsöffnungen 14 im zusammengeklappten Zustand der Manschette M sind. Auf diese Weise ist es möglich einen chirurgischen Faden 28 durch die Öffnungen 14, 14' der von der Manschette M umfassten Manschettenelektrode CE zu führen. Hierdurch kann die Metallring ummantelte Befestigungsöffnung 14 der Manschettenelektrode CE durch die innerhalb der Manschette M eingebrachte Befestigungsöffnung 14' entlastet werden. Vorzugsweise ist die Manschette M aus einem stabilen Kunststoffmaterial gefertigt, beispielsweise aus Parylen. Zur weiteren Steigerung der Festigkeit, kann die Mo und Mu auch aus einem Polymerhybrid bestehen (z.B. Parylen (innen) und Silikongummi aussen). Dieser Hybrid hat den Vorteil, dass die Stabilität des Parylen mit der Reißfestigkeit des Silikons kombiniert wird. In einer bevorzugten Ausführungsform sind die Befestigungsöffnungen 14' innerhalb der Manschette M durch entsprechende Materialaufdickung verstärkt ausgeführt.

In Manschettenoberteil Mo sind Öffnungsfenster 29 eingebracht, die einen freien Zugang zu den Referenzelektrodenflächen 12 gewährleisten. In Figur 4e ist ein diesbezüglicher Querschnitt durch das von der Manschette M umfasste Trägersubstrat 1 dargestellt, an dessen Oberseite Referenzelektrodenflächen 12 eingebracht sind, die durch die innerhalb des Manschettenoberteils Mo eingebrachten Öffnungsfenster 29 frei zugänglich bleiben. Vorzugsweise umfassen die Öffnungsfenster 29 die Referenzelektrodenflächen 12 mit einer schräg abfallenden Begrenzungsflanke 29', so dass dafür gesorgt ist, dass die Referenzelektrodenflächen 29 ganzflächig mit umliegendem Gewebe in Körperkontakt treten können.

Um sicherzustellen, dass die Manschette M in einem geschlossenen Zustand verbleibt, sind zwischen dem Manschettenober- und -unterteil Mo, Mu Verriegelungsstrukturen V angeordnet, die beispielsweise aus einem Pin 30 und einer gegenliegend angeordneten Vertiefung 31 bestehen, siehe Figuren 4c und f. Beim Zusammenklappen des Manschettenober- und -unterteils fügen sich die Pins 30 kraftbeaufschlagt in die entsprechenden Vertiefung 31, in der der Pin 31 jeweils reibschlüssig, dauerhaft gehalten wird. In Figur 4f ist der geschlossene Zustand einer Verriegelungsstruktur V illustriert. Hierbei ragt der am Manschettenoberteil Mo angebrachte Pin 30 durch eine entsprechende im Trägersubstrat 1 eingebrachte Öffnung und mündet endseitig innerhalb der Vertiefung 31 des Manschettenunterteils Mu. Selbstverständlich sind alternative Ausgestaltungsformen für die Verriegelungsstrukturen denkbar, beispielsweise in Form geeignet ausgebildeter Rastmechanismen.

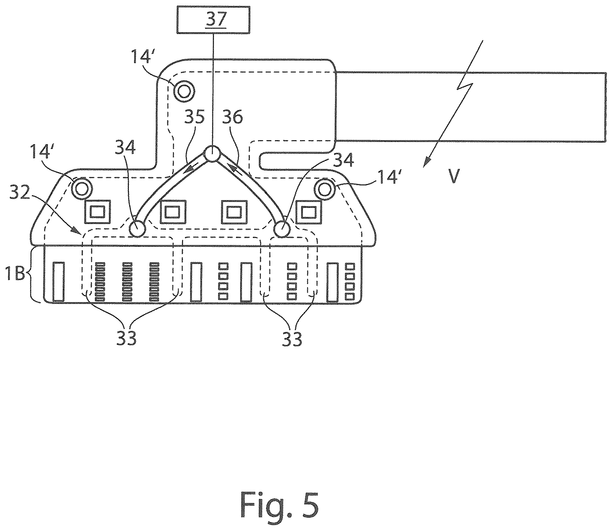

In Figur 5 ist eine weitere Ausführungsform illustriert, die eine erleichterte Implantation der lösungsgemäß ausgebildeten Manschettenelektrode CE ermöglicht. Innerhalb des Trägersubstrats 1 ist ein Fluidkanalsystem 32 eingearbeitet, das vollständig vom Trägersubstrat 1 umfasst ist. Das Fluidkanalsystem 32 erstreckt sich im Wesentlichen im Bereich des Trägersubstratbereiches 1B, der aufgrund einer materialinhärenten Vorspannung ohne äußere Krafteinwirkung im Wege einer autonomen Selbstaufwicklung die Gestalt eines Gradzylinders annimmt. Wird hingegen das Fluidkanalsystem 32 mit einem Fluid, vorzugsweise Wasser gefüllt, so vermag der sich längs des Fluidkanalsystems 32 ausbildende Wasserdruck den Trägersubstratbereich 1b entgegen der materialinhärenten Wickelkräfte eben aufzuspannen. Hierzu verfügt das Fluidkanalsystem 32 über sich in Umfangsrichtung der Mantelfläche des sich selbständig ausbildenden Gradzylinders verlaufende Fluidkanaläste 33, die im befüllten Zustand die erforderliche Streckung des Trägersubstratbereiches 1B erzwingen.

Zur Befüllung des Fluidkanalsystems 32 sind wenigstens zwei Kanalöffnungen 34 innerhalb des Trägersubstrats 1 vorgesehen, deren Größe und Anordnung derart bemessen sind, dass sie fluiddicht an Ein- und Austrittsöffnungen von innerhalb der Manschette M verlaufenden Fluidzu- bzw. -ableitungen 35, 36 münden. Die innerhalb der Manschette M verlaufenden Zu- bzw. Ableitungen 35, 36 sind fluidisch mit einem Fluidsteuersystem 37 verbunden, das von einem Operateur betätigbar ist.

Im Falle einer Implantation ist das Fluidkanalsystem 32 mit einem Fluid befüllt, wodurch der Trägersubstratbereich 1B gestreckt ist. In diesem Zustand platziert der Operateur die Manschettenelektrode CE exakt an einer vorgegebenen Stelle längs des Nervenbündels. Anschließend erfolgt die Entleerung des Fluidkanalsystems 32 durch den Operateur, wodurch sich der Trägersubstratbereich 1B selbsttätig um das Nervenfaserbündel wickelt. Als letzter Schritt wird die Manschettenelektrode CE mit einem chirurgischen Faden durch die in der Manschette vorgesehenen Befestigungsöffnungen 14' am umliegenden Gewebe fixiert.

In einer vorteilhaften Ausgestaltung des vorstehenden Fluidkanalsystems 32 ist es denkbar dieses mit einem Formgedächtnismetall- Formgedächtnispolymer zu füllen. Zu Zwecken der Aktivierung sind die Kanalöffnungen 34 mit metallisierten Kontakten versehen, über die eine elektrische Spannung längs der Zuleitungen 35, 36 zur Entfaltung der implantierbaren Elektrodenanordnung CE über ein entsprechend modifiziertes Steuergerät 37 anlegbar ist, bis die Elektrode letztlich platziert ist.

### Bezugszeichenliste

- 1: Trägersubstrat
- 1': Trägersubstratoberfläche
- 1B: Trägersubstratbereich
- 2: erste Elektrodenanordnung
- 3: erste Elektrodenstrukturen
- 4: erste Elektrodenflächen
- 4a: axiale Erstreckung der ersten Elektrodenflächen
- 4U: in Umfangsrichtung orientierte Erstreckung der ersten Elektrodenflächen
- 5: erste Elektrodenstreifen
- 6, 6': Signaldetektor und -generator
- 7: zweite Elektrodenanordnung
- 8: zweite Elektrodenstreifen
- 9: zweite Elektrodenflächen
- 9a: axiale Erstreckung der zweiten Elektrodenflächen
- 9U: in Umfangsrichtung orientierte Erstreckung der zweiten Elektrodenflächen
- 10: Lichtwellenleiteranordnung
- 11: Lichtwellenleiteröffnungen
- 12: Referenzelektrodenflächen, EKG-Elektrodenflächen
- 13: zweite Elektrodenstruktur
- 14: Befestigungsöffnungen
- 14': Befestigungsöffnung
- 15: Öffnung
- 16: Elektrodenstreifenoberfläche
- 17: Basisplatte
- 18: Oberseite
- 19: Unterseite
- 20: Strukturelement
- 21: Oberflächenbereich
- 22,: Haftvermittlerschicht
- 22': Haftvermittlerschichtanordnung
- 23: Zweiter Oberflächenbereich
- 24: dritter Oberflächenbereich
- 25: Filmscharniergelenk
- 26: Vertiefung
- 27: Vertiefung
- 28: Chirurgischer Faden
- 29: Öffnungsfenster
- 29': Begrenzungsflanke
- 30: Pin
- 31: Vertiefung
- 32: Fluidkanalsystem
- 33: Fluidkanaläste
- 34: Kanalöffnung
- 35: Zuleitung, innerhalb der Manschette
- 36: Ableitung, innerhalb der Manschette
- 37: Fluidsteuersystem
- CE: Manschettenelektrode
- L: Leiterbahn
- V: Verbindungsstruktur
- U: Umfangsrichtung
- A: axiale Richtung
- M: Manschette
- Mo: Manschettenoberteil
- Mu: Manschettenunterteil
- NF: Nervenfaser
- NFB: Nervenfaserbündel
- G: Gehirn
- H: Herz
- LI: Lichtwellenleiter
- LQ: Lichtquelle(n)
- LA: Längsachse des Strukturelementes
- KO: Geometrische Konstellationen
- V: Verriegelungsstruktur

## Patentansprüche

1. Implantierbare Elektrodenanordnung zur ortsselektiven Erfassung neuronaler elektrischer Signale, die sich längs wenigstens einer in einem Nervenfaserbündel enthaltenden Nervenfaser ausbreiten, sowie zur selektiven elektrischen Stimulation der wenigstens einen Nervenfaser, mit einem biokompatiblen Trägersubstrat (1), das zumindest einen Trägersubstratbereich aufweist, der um das Nervenfaserbündel manschettenartig anlegbar ist und eine im implantierten Zustand dem Nervenfaserbündel zugewandt orientierte, geradzylinderförmige Trägersubstratoberfläche (1') aufweist, die eine axiale (a) sowie eine in Umfangsrichtung (U) orientierte Erstreckung besitzt und an der eine erste Elektrodenanordnung (2) angebracht ist, die
- in axialer Abfolge wenigstens drei erste Elektrodenstrukturen (3) mit jeweils wenigstens zwei in Umfangsrichtung angeordneten ersten Elektrodenflächen (4), sowie
- wenigstens zwei axial zueinander beabstandete, sich in Umfangsrichtung erstreckende und jeweils eine Ringform annehmende erste Elektrodenstreifen (5) umfasst, die die wenigstens drei Elektrodenstrukturen axial beidseitig einschließen, und die mit einem Signaldetektor und -generator (6) verbindbar ist, **dadurch gekennzeichnet, dass** in axialer Abfolge auf der dem Nervenfaserbündel zugewandten geradzylinderförmigen Trägersubstratoberfläche (1') axial neben der ersten Elektrodenanordnung (2) wenigstens eine zweite Elektrodenanordnung (7) angeordnet ist, die
- wenigstens zwei axial voneinander beabstandete, sich in Umfangsrichtung (U) erstreckende und jeweils eine Ringform annehmende zweite Elektrodenstreifen (8) sowie
- axial zwischen den wenigstens zwei zweiten Elektrodenstreifen (8) wenigstens eine, jeweils wenigstens zwei in Umfangsrichtung (U) gleichverteilt angeordnete zweite Elektrodenflächen (9) umfassende, zweite Elektrodenstruktur (13) umfasst und
wobei die zweite Elektrodenanordnung (7) zumindest mit dem Signalgenerator (6) oder einem weiteren Signalgenerator (6') verbindbar ist.

2. Elektrodenanordnung nach Anspruch 1,
**dadurch gekennzeichnet, dass** im implantierten Zustand die ersten und zweiten Elektrodenflächen (4, 9) in Umfangsrichtung (U) jeweils längs einer virtuellen Kreislinie gleichverteilt angeordnet sind.

3. Elektrodenanordnung nach Anspruch 1 oder 2,
**dadurch gekennzeichnet, dass** die ersten und zweiten Elektrodenflächen (4, 9) jeweils eine axiale (4a, 9a) und eine in Umfangsrichtung (4U, 9U) orientierte Erstreckung aufweisen,
dass die Erstreckungen (4a, 4U) der ersten Elektrodenflächen (4) jeweils identisch sind,
dass die Erstreckungen (9a, 9U) der zweiten Elektrodenflächen (9) jeweils identisch sind, und
dass die in Umfangsrichtung orientierte Erstreckung (9U) der zweiten Elektrodenflächen (9) größer ist als die in Umfangsrichtung orientierte Erstreckung (4U) der ersten Elektrodenflächen (4).

4. Elektrodenanordnung nach Anspruch 3,
**dadurch gekennzeichnet, dass** die axialen Erstreckungen (4a, 4U) der ersten und zweiten Elektrodenflächen (4, 9) gleich sind.

5. Elektrodenanordnung nach einem der Ansprüche 1 bis 4,
**dadurch gekennzeichnet, dass** die Trägersubstratoberfläche (1'), auf der die erste und zweite Elektrodenanordnung (2, 7) aufgebracht ist, einstückig zusammenhängend, d.h. unterbrechungsfrei, ausgebildet ist.

6. Elektrodenanordnung nach einem der Ansprüche 1 bis 5,
**dadurch gekennzeichnet, dass** der axiale Abstand zwischen den ersten Elektrodenstreifen (5) größer oder gleich dem axialen Abstand zwischen den zweiten Elektrodenstreifen (8) gewählt ist, und
dass der axiale Abstand zwischen den zweiten Elektrodenstreifen zwischen 0,5 cm und 3 cm, vorzugsweise zwischen 0,75 cm und 1,25 cm misst.

7. Elektrodenanordnung nach einem der Ansprüche 1 bis 6,
**dadurch gekennzeichnet, dass** die Form und Größe der ersten und zweiten Elektrodenstreifen (5, 8) identisch sind, und
dass die Flächengrößen jeweils der ersten und zweiten Elektrodenfläche (4, 9) kleiner sind als die Flächengröße jeweils der ersten oder zweiten Elektrodenstreifen (5, 8), vorzugsweise kleiner als ein Viertel der Flächengröße der ersten oder zweiten Elektrodenstreifen (5, 8).

8. Elektrodenanordnung nach einem der Ansprüche 1 bis 7,
**dadurch gekennzeichnet, dass** die ersten Elektrodenflächen (4) aus einem metallischen Material bestehen, das über eine höhere Ladungsübertragungskapazität verfügt als ein Material aus dem die zweiten Elektrodenflächen (9) bestehen.

9. Elektrodenanordnung nach Anspruch 8,
**dadurch gekennzeichnet, dass** das metallische Material der ersten Elektrodenflächen (4) Iridium-Oxid ist, und/oder
dass das Material der zweiten Elektrodenflächen (9) ein metallisches Material, vorzugsweise Platin, oder ein elektrisch leitfähiges Polymer ist.

10. Elektrodenanordnung nach einem der Ansprüche 1 bis 9,
**dadurch gekennzeichnet, dass** die erste und zweite Elektrodenanordnung (2, 7) jeweils als Tripolar-Elektrodenanordnung betreibbar ist, d.h. die ersten und zweiten Elektrodenstreifen (5, 8) sind jeweils gegenpolig zur ersten und zweiten Elektrodenstruktur (3, 13) polarisierbar.

11. Elektrodenanordnung nach einem der Ansprüche 1 bis 10,
**dadurch gekennzeichnet, dass** im Bereich der zweiten Elektrodenanordnung (7) wenigstens eine optische Lichtwellenleiteranordnung (10) vorgesehen ist, die wenigstens zwei in Umfangsrichtung (U) verteilt angeordnete, separate Lichtwellenleiteröffnungen (11) umfasst.

12. Elektrodenanordnung nach Anspruch 11,
**dadurch gekennzeichnet, dass** die wenigstens zwei separaten Lichtwellenleiteröffnungen (11) längs einer virtuellen Kreislinie gleichverteilt angeordnet sind, und
dass die Lichtwellenleiteröffnungen (11) jeweils eine axiale (11a) und eine in Umfangsrichtung (11U) orientierte Erstreckung besitzen, die jener Erstreckungen (9a, 9U) der zweiten Elektrodenflächen (9) entsprechen.

13. Elektrodenanordnung nach einem der Ansprüche 1 bis 12,
**dadurch gekennzeichnet, dass** die ersten Elektrodenflächen (4) sowie die ersten Elektrodenstreifen (5) der ersten Elektrodenanordnung (2) sowie die zweiten Elektrodenflächen (9) sowie die zweiten Elektrodenstreifen (8) der zweiten Elektrodenanordnung (7) jeweils derart an der Trägersubstratoberfläche (1') angebracht sind, so dass sie die Trägersubstratoberfläche (1') nicht überragen.

14. Elektrodenanordnung nach einem der Ansprüche 1 bis 13,
**dadurch gekennzeichnet, dass** das Trägersubstrat (1) aus wenigstens einem biokompatiblen Polymer gefertigt ist und zumindest bereichsweise an der dem Nervenfaserbündel zugewandten geradzylinderförmigen Trägersubstratoberfläche (1') einen Inflammationsreaktionen inhibierenden Wirkstoff aufweist.

15. Elektrodenanordnung nach einem der Ansprüche 1 bis 14,
**dadurch gekennzeichnet, dass** wenigstens der Signaldetektor und -generator (6), der optional vorgesehene zweite Signaldetektor (6') und/oder eine elektrische Energieversorgungseinheit separat zum Trägersubstrat (1) innerhalb einer kapselartigen Einhausung hermetisch eingefasst oder integrale Bestandteile des Trägersubstrats (1) sind.

16. Elektrodenanordnung nach einem der Ansprüche 1 bis 15,
**dadurch gekennzeichnet, dass** das Trägersubstrat (1) biokompatibles Polymer enthält.

17. Elektrodenanordnung nach Anspruch 16,
**dadurch gekennzeichnet, dass** die ersten und/oder zweiten Elektrodenstreifen (5, 8) jeweils über wenigstens eine lokale Öffnung verfügen, und dass die ersten und/oder zweiten Elektrodenstreifen (5, 8) derart mit der Trägersubstratoberfläche (1') flächig verbunden sind, sodass das Polymer die wenigstens eine Öffnung wenigstens teilweise durchdringt.

18. Elektrodenanordnung nach einem der Ansprüche 1 bis 17,
**dadurch gekennzeichnet, dass** rückseitig zur Trägersubstratoberfläche (1') an dem Trägersubstrat (1) wenigstens zwei Referenzelektrodenflächen (12) angebracht sind.

19. Elektrodenanordnung nach einem der Ansprüche 1 bis 18,
**dadurch gekennzeichnet, dass** das biokompatible Trägersubstrat (1) im Bereich der dem Nervenfaserbündel zugewandt orientierten, geradzylinderförmigen Trägersubstratoberfläche (1') jeweils axial sich gegenüberliegende Randbereiche aufweist, an denen das Trägersubstrat (1) über eine größere Substratdicke verfügt als im übrigen Trägersubstratbereich, und
dass die Randbereiche über abgerundete Randkanten verfügen.

20. Elektrodenanordnung nach einem der Ansprüche 1 bis 19,
**dadurch gekennzeichnet, dass** das biokompatible Trägersubstrat (1) in einem Trägersubstratbereich, der nicht um das Nervenfaserbündel manschettenartig anlegbar ist, wenigstens eine das Trägersubstrat vollständig durchsetzende Befestigungsöffnung (14) aufweist.

21. Elektrodenanordnung nach Anspruch 20,
**dadurch gekennzeichnet, dass** die wenigstens eine Befestigungsöffnung (14) zumindest bereichsweise mit einem metallischen Material ummantelt ist.

22. Elektrodenanordnung nach Anspruch 16,
**dadurch gekennzeichnet, dass** die ersten und/oder zweiten Elektrodenstreifen (5, 8) jeweils eine metallische Basisplatte (17) mit einer ebenen Ober- (18) und Unterseite (19) aufweisen, mit wenigstens einem die Oberseite (18) orthogonal, lokal überragenden Strukturelement (20),
dass die ebene Oberfläche (18) der metallischen Basisplatte (17) parallel zur Trägersubstratoberfläche (1') orientiert ist, und
dass die metallische Basisplatte (17) vollständig von dem biokompatiblen Polymer mittel- und/oder unmittelbar umschlossen ist mit Ausnahme eines ersten Oberflächenbereiches (21) des wenigstens einen Strukturelements (20), der der Trägersubstratoberfläche (1') zugewandt orientiert ist und diese nicht überragt.

23. Elektrodenanordnung nach Anspruch 22,
**dadurch gekennzeichnet, dass** zumindest zwischen der Unterseite (19) der metallischen Basisplatte (17) und dem biokompatiblen Polymer des Trägersubstrats (1) eine Haftvermittlerschicht (22) oder eine Haftvermittlerschichtanordnung (22') eingebracht ist.

24. Elektrodenanordnung nach Anspruch 22 oder 23,
**dadurch gekennzeichnet, dass** der erste Oberflächenbereich (21) des wenigstens einen Strukturelements (20) oder eine dem ersten Oberflächenbereich (21) zugeordnete Ebene (E) parallel zur Trägersubstratoberfläche (1') orientiert ist, dass der erste Oberflächenbereich (21) von Seiten der Trägersubstratoberfläche (1') frei zugänglich angeordnet ist und dass das wenigstens eine Strukturelement (20) einstückig mit der metallischen Basisplatte (17) verbunden ist.

25. Elektrodenanordnung nach einem der Ansprüche 22 bis 24,
**dadurch gekennzeichnet, dass** eine Vielzahl an der Oberseite (18) der metallischen Basisplatte (17) nach einem geometrischen Muster (KO) angeordnete und identisch ausgebildete Strukturelemente (20) vorgesehen ist.

26. Elektrodenanordnung nach einem der Ansprüche 22 bis 25,
**dadurch gekennzeichnet, dass** das wenigstens eine Strukturelement (20) säulen-, rippen-, hülsen oder stegartig ausgebildet ist.

27. Elektrodenanordnung nach einem der Ansprüche 23 bis 25,
**dadurch gekennzeichnet, dass** das wenigstens eine Strukturelement (20) eine orthogonal zur Oberseite der metallischen Basisplatte (17) orientierte Längserstreckung (LA) aufweist, längs der das Strukturelement (20) wenigstens einen zweiten Oberflächenbereich (23) vorsieht, der parallel zur Oberseite (18) der metallischen Basisplatte (17) orientiert ist und auf dem die Haftvermittlerschicht (22) oder eine Haftvermittlerschichtanordnung (22') aufgebracht ist, und
dass der zweite Oberflächenbereich (23) vom ersten Oberflächenbereich (18) beabstandet angeordnet und mittelbar vollständig von dem biokompatiblen Polymer umgeben ist.

28. Elektrodenanordnung nach einem der Ansprüche 1 bis 27,
**dadurch gekennzeichnet, dass** zumindest in einem die Trägersubstratoberfläche (1') nicht enthaltenden Teilbereich des Trägersubstrats (1) das Trägersubstrat (1) von einer Manschette (M) umfasst ist.

29. Elektrodenanordnung nach Anspruch 28 und 20 oder 21,
**dadurch gekennzeichnet, dass** die Manschette (1) über eine Manschettenober- (Mo) und -unterseite (Mu) verfügt, die einstückig gelenkig miteinander verbunden sind, und
dass die Manschettenober- (Mo) und -unterseite (Mu) jeweils Befestigungsöffnungen (14') aufweisen, die deckungsgleich zu den Befestigungsöffnungen (14) des Trägersubstrats (1) angeordnet sind im Zustand der das Trägersubstrat (1) umfassenden Manschette (M).

30. Elektrodenanordnung nach einem der Ansprüche 16 bis 29,
**dadurch gekennzeichnet, dass** das Trägersubstrat (1) eine orthogonal zur Trägersubstratoberfläche (1') orientierte Substratdicke aufweist, und dass die Basisplatte (17) mittig zur Substratdicke angeordnet ist.

## Claims

1. Implantable electrode device for the recording at selected positions of neuronal electrical signals which spread out along a nerve fibre contained in a bundle of nerve fibres, as well as for the selective electrical stimulation of the at least one nerve fibre, with a biocompatible carrier substrate (1) which comprises at least one carrier substrate area which can be applied in a cuff-like manner around the nerve fibre bundle and a straight cylindrical carrier substrate surface (1'), which is orientated facing the nerve fibre bundle when implanted and which has an axial (a) extension as well as an extension oriented in the circumferential direction (U) and on which a first electrode device (2), is applied which
- axially comprises at least three first electrode structures (3) each with at least two first electrode surfaces (4) arranged in the circumferential direction and
- at least two axially spaced first electrode strips (5) extending in the circumferential direction and each taking on an annular shape, which on both sides axially encompass the at least three electrode structures and which is connectable to a signal detector and generator (6),
**characterised in that** in axial sequence on the straight cylindrical carrier substrate surface (1') facing the nerve fibre bundle, arranged axially next to the first electrode device (2) is at least one second electrode device (7), which comprises
- at least two axially spaced second electrode strips (8) extending in the circumferential direction and each taking on an annular shape as well as
- axially between the two second electrode strips (8) at least one second electrode structure (13) comprising at least two second electrode surfaces (9) arranged evenly distributed in the circumferential direction (U) and
wherein the second electrode device is connectable at least with the signal generator (6)
or a further signal generator (6').

2. Electrode device according to claim 1
**characterised in that** in the implanted state the first and second electrode surfaces (4, 9) are each arranged evenly distributed along a virtual circle line in the circumferential direction (U).

3. Electrode device according to claim 1 or 2
**characterised in that** the first and second electrode surfaces (4, 9) each have an axial (4a, 9a) extension and an extension orientated in the circumferential direction (4U, 9U)
**in that** the extension (4a, 4U) of the first electrode surfaces (4) are each identical,
**in that** the extensions (9a, 9U) of the second electrode surfaces (9) are each identical
and **in that** the extension (9U) orientated in the circumferential direction of the second electrode surfaces (9) is larger than the extension (4U) of the first electrode surfaces (4) orientated in the circumferential direction.

4. Electrode device according to claim 3
**characterised in that** the axial extension (4a, 4U) of the first and second electrode surfaces (4, 9) are the same.

5. Electrode device according to any one of claims 1 to 4
**characterised in that** the carrier substrate surface (1'), applied on the first and second electrode device (2, 7) is designed in one piece, contiguously, i.e. without interruptions.

6. Electrode device according to any one of claims 1 to 5
**characterised in that** the axial spacing between the first electrode strips (5) is selected to be greater than or equal to the axial spacing between the second electrode strips (8), and **in that** the axial spacing between the second electrode strips measures between 0.5 cm and 3 cm, preferably between 0.75 cm and 1.25 cm.

7. Electrode device according to any one of claims 1 to 6
**characterised in that** the shape and size of the first and second electrode strips are identical and
**in that** the surface areas of the first and second electrode surfaces (4, 9) are smaller than the surface areas of the first or second electrode strips (5, 8) respectively, preferably smaller than a quarter of the surface areas of the first or second electrode strips (5, 8).

8. Electrode device according to any one of claims 1 to 7
**characterised in that** the first electrode surfaces (4) comprise a metallic material which has a greater charge transfer capacity than a material of which the second electrode surfaces (9) consists.

9. Electrode device according to claim 8,
**characterised in that** the metallic material of the first electrode surfaces (4) is iridium oxide and/or in that the material of the second electrode surfaces (9) is a metallic material, preferably platinum, or an electrically conductive polymer.

10. Electrode device according to any one of claims 1 to 9
**characterised in that** both the first and second electrode device (2, 7) can be operated as a tripolar electrode device, i.e. the first and second electrode strips (5, 8) can each be polarised to the opposite pole of the first and second electrode structure (3, 13) .

11. Electrode device according to any one of claims 1 to 10
**characterised in that** in the area of the second electrode device (7) at least one optical waveguide device (10) is provided, which comprises at least two optical waveguide openings arranged in the circumferential direction (U).

12. Electrode device according to claim 11
**characterised in that** the at least two separate optical waveguide openings (11) are arranged evenly distributed along a virtual circle line and **in that** the optical waveguide openings (11) each have an axial (11a) extension and an extension orientated in the circumferential direction (11U), which correspond to the extension (9a, 9U) of the second electrode surfaces (9).

13. Electrode device according to any one of claims 1 to 12
**characterised in that** the first electrode surfaces (4) as well as the first electrode strips (5) of the first electrode device (2) as well as the second electrode surfaces (9) as well as the second electrode strips (8) of the second electrode device (7) are applied to the carrier substrate surface (1') in such a way that they do not project beyond the carrier substrate surface (1').

14. Electrode device according to any one of claims 1 to 13
**characterised in that** the carrier substrate (1) is made of at least one biocompatible polymer and at least in part on the straight cylindrical carrier substrate surface (1') facing the nerve fibre bundle comprises an active substance that inhibits inflammatory reactions.

15. Electrode device according to any one of claims 1 to 14
**characterised in that** at least the signal detector and generator (6), the optionally provided second signal detector (6') and/or an electrical energy supply unit are hermetically encompassed separate from the carrier substrate (1) within a capsule-like casing or form an integral part of the carrier substrate (1).

16. Electrode device according to any one of claims 1 to 15
**characterised in that** the carrier substrate (1) contains biocompatible polymer.

17. Electrode device according to claim 16
**characterised in that** the first and/or the second electrode strips (5, 8) each have at least one local opening and **in that** the first and/or second electrode strips (5, 8) are two-dimensionally connected to the carrier substrate surface (1') in such a way that the polymer at least partially penetrates the at least one opening.

18. Electrode device according to any one of claims 1 to 17
**characterised in that** on the rear side of the carrier substrate surface (1') at least two reference electrode surfaces (12) are applied to the carrier substrate (1).

19. Electrode device according to any one of claims 1 to 18
**characterised in that** the biocompatible carrier substrate (1) in the area of the straight cylindrical carrier substrate surface (1') facing the nerve fibre bundle has axially opposite marginal areas at which the carrier substrate (1) has a greater substrate thickness than in the remaining carrier substrate area and **in that** the marginal area have rounded margin edges.

20. Electrode device according to any one of claims 1 to 19
**characterised in that** the biocompatible carrier substrate (1), in an area of the carrier substrate which cannot be applied around the nerve fibre bundle in a cuff-like manner, comprises a fastening opening (14) which completely penetrates the carrier substrate.

21. Electrode device according to claim 20
**characterised in that** the at least one fastening opening (14) at least in parts is surrounded by a metallic material.

22. Electrode device according to claim 16
**characterised in that** the first and/or the second electrode strips (5, 8) each comprise a metallic base place (17) with a flat upper (18) and lower side (19), with a least one structural element (20) locally orthogonally, projecting beyond the upper side (18),
**in that** the flat upper surface (18) of the metallic base plate (17) is orientated in parallel to the carrier substrate upper surface (1') and
**in that** the metallic base plate (17) is completely directly and/or indirectly surrounded by the biocompatible polymer with the exception of a first upper surface area (21) of the at least one structural element (20), which is orientated facing the carrier substrate surface (1') and does not project beyond it.

23. Electrode device according to claim 22
**characterised in that** a layer of bonding agent (22) or a bonding agent layering device (22') is introduced at least between the underside (19) of the metallic base plate (17) and the biocompatible polymer of the carrier substrate (1).

24. Electrode device according to claim 22 or 23
**characterised in that** the first surface area (21) of the at least one structural element (20) or a plane (E) assigned to the first surface area (21) is oriented in parallel to the carrier substrate surface (1'), in the first surface area (21) is arranged to be freely accessible from the carrier substrate surface (1') and that at least one structural element (20) is connected in one piece to the metallic base plate (17) .

25. Electrode device according to any one of claims 22 to 24
**characterised in that** a plurality of structural elements (20) is provided which are arranged in a geometric pattern (KO) on the upper side (18) of the metallic base plate (17) and are identical in design.

26. Electrode device according to any one of claims 22 to 25
**characterised in that** the at least one structural element (20) is designed in a column, rib, sleeve or web-like manner.

27. Electrode device according to any one of claims 23 to 25
**characterised in that** the at least one structural element (20) has a longitudinal extension (LA), orientated orthogonally to the upper side of the metallic base plate (17), along which the structural element (20) envisages at least one surface area (23) which is orientated in parallel to the upper side (18) of the base plate (17) and on which the bonding agent layer or bonding agent layering device (22') is applied and **in that** the second surface area (23) is arranged at a distance from the first surface area (18) and is completely surrounded by the biocompatible polymer.

28. Electrode device according to any one of claims 1 to 27
**characterised in that** at least in a partial area of the carrier substrate (1) not containing the carrier substrate surface (1') the carrier substrate (1) is encompassed by a cuff (M).

29. Electrode device according to claim 28 and 20 or 21,
**characterised in that** the cuff (1) has a cuff upper side (Mo) and underside (Mu) which are connected to each other in one piece in an articulated manner
and
**in that** the cuff upper side (Mo) and underside (Mu) each have fastening openings (14') which are arranged congruently with the fastening openings (14) of the carrier substrate (1) in the state in which the cuff (M) encompasses the carrier substrate (1).

30. Electrode device according to any one of claims 16 to 29 **characterised in that** the carrier substrate (1) has a substrate thickness orientated orthogonally to the carrier substrate surface (1') and **in that** the base plate (17) is arranged centred on the substrate thickness.

## Revendications

1. Système d'électrodes implantable pour la saisie localement sélective de signaux électriques neuronaux, qui se propagent le long d'au moins une fibre nerveuse contenue dans un faisceau de fibres nerveuses, ainsi que pour la stimulation électrique sélective d'au moins une fibre nerveuse, avec un substrat de support biocompatible (1), qui comporte au moins une zone de substrat de support qui peut être appliquée sous la forme d'une manchette autour du faisceau de fibres nerveuses et comporte une surface de substrat de support (1') cylindrique droite orientée à l'état implanté vers le faisceau de fibres nerveuses, qui possède une extension axiale (a) ainsi qu'une extension orientée dans la direction périphérique (U) et à laquelle est appliqué un premier système d'électrodes (2), qui comprend
- en succession axiale au moins trois premières structures d'électrodes (3) avec respectivement au moins deux premières surfaces d'électrodes (4) disposées dans la direction périphérique, ainsi qu'
- au moins deux premières bandes d'électrodes (5) s'étendant à distance axialement l'une de l'autre et adoptant respectivement une forme annulaire, qui comprennent axialement des deux côtés au moins trois structures d'électrodes, et, qui peut être relié à un détecteur et générateur de signaux (6), **caractérisé en ce que** dans la succession axiale au moins un deuxième système d'électrodes (7) est disposé axialement près du premier système d'électrodes (2) sur la surface de substrat de support (1') cylindrique droite tournée vers le faisceau de fibres nerveuses, qui comprend
- au moins deux deuxièmes bandes d'électrodes (8) axialement à distance l'une de l'autre, s'étendant dans la direction périphérique (U) et adoptant respectivement une forme annulaire, ainsi
- qu'au moins une deuxième structure d'électrodes (13) disposée axialement entre au moins les deux deuxièmes bandes d'électrodes (8), comprenant respectivement au moins deux surfaces d'électrodes (9) uniformément réparties dans la direction périphérique (U), et
le deuxième système d'électrodes (7) pouvant être relié au moins au générateur de signaux (6) ou à un autre générateur de signaux (6').

2. Système d'électrodes selon la revendication 1, **caractérisé en ce qu'**à l'état implanté, les premières et deuxièmes surfaces d'électrodes (4,9) sont respectivement disposées dans la direction périphérique (U), uniformément réparties le long d'une ligne circulaire virtuelle.

3. Système d'électrodes selon la revendication 1 ou 2, **caractérisé en ce que** les premières et deuxièmes surfaces d'électrodes (4,9) comportent respectivement une extension axiale (4a,9a) et une extension orientée dans la direction périphérique (4U,9U),
**en ce que** les extensions (4a,4U) des premières surfaces d'électrodes (4) sont respectivement identiques,
**en ce que** les extensions (9a,9U) des deuxièmes surfaces d'électrodes (9) sont respectivement identiques, et
**en ce que** l'extension (9U) des deuxièmes surfaces d'électrodes (9),orientée dans la direction périphérique est plus grande que l'extension (4U) des premières surfaces d'électrodes (4), orientée dans la direction périphérique.

4. Système d'électrodes selon la revendication 3, **caractérisé en ce que** les extensions axiales (4a,4U) des premières et deuxièmes surfaces d'électrodes (4,9) sont identiques.

5. Système d'électrodes selon l'une quelconque des revendications 1 à 4, **caractérisé en ce que** la surface de substrat de support (1') est appliquée au premier et au deuxième système d'électrodes (2,7) et est constituée en une seule pièce, en continu, c'est-à-dire sans interruption.

6. Système d'électrodes selon l'une quelconque des revendications 1 à 5, **caractérisé en ce que** la distance axiale entre les premières bandes d'électrodes (5) est choisie plus grande ou égale à la distance axiale entre les deuxièmes bandes d'électrodes (8) et **en ce que** la distance axiale entre les deuxièmes bandes d'électrodes mesure entre 0,5 cm et 3 cm, de préférence entre 0,75 cm et 1,25 cm.

7. Système d'électrodes selon l'une quelconque des revendications 1 à 6, **caractérisé en ce que** la forme et la taille des premières et deuxièmes bandes d'électrodes (5,8) sont identiques et **en ce que** les tailles de surface respectivement de la première et de la deuxième surface d'électrodes (4,9) sont plus petites que la taille de surface respectivement de la première et de la deuxième bande d'électrodes (5,8), de préférence plus petite qu'un quart de la taille de surface des premières ou deuxièmes bandes d'électrodes (5,8).

8. Système d'électrodes selon l'une quelconque des revendications 1 à 7, **caractérisé en ce que** les premières surfaces d'électrodes (4) sont composées d'un matériau métallique, qui dispose d'une capacité de transfert de charge plus élevée qu'un matériau dans lequel sont composées les deuxièmes surfaces d'électrodes (9).

9. Système d'électrodes selon la revendication 8, **caractérisé en ce que** le matériau métallique des premières surfaces d'électrodes (4) est de l'oxyde d'iridium et/ou **en ce que** le matériau des deuxièmes surfaces d'électrodes (9) est un matériau métallique, de préférence du platine, ou un polymère électro-conducteur.

10. Système d'électrodes selon l'une quelconque des revendications 1 à 9, **caractérisé en ce que** le premier et le deuxième système d'électrodes (2,7) peuvent être utilisés respectivement en tant que système d'électrodes tripolaire, c'est-à-dire que les premières et deuxièmes bandes d'électrodes (5,8) peuvent être respectivement polarisées en polarité opposée par rapport à la première et la deuxième structure d'électrodes (3,13).

11. Système d'électrodes selon l'une quelconque des revendications 1 à 10, **caractérisé en ce que** dans la zone du deuxième système d'électrodes (7) au moins un système à fibres optiques (10) est prévu, qui comprend au moins deux ouvertures pour fibres optiques (11) séparées disposées réparties dans la direction périphérique (U).

12. Système d'électrodes selon la revendication 11, **caractérisé en ce qu'**au moins deux ouvertures pour fibres optiques séparées (11) sont disposées uniformément réparties le long d'une ligne circulaire virtuelle et **en ce que** les ouvertures pour fibres optiques (11) possèdent respectivement une extension axiale (11a) et une orientée dans la direction périphérique (11U), qui correspondent à chacune des extensions (9a,9U) des deuxièmes surfaces d'électrodes (9) .

13. Système d'électrodes selon l'une quelconque des revendications 1 à 12, **caractérisé en ce que** les premières surfaces d'électrodes (4) ainsi que les premières bandes d'électrodes (5) du premier système d'électrodes (2) ainsi que les deuxièmes surfaces d'électrodes (9) et les deuxièmes bandes d'électrodes (8) du deuxième système d'électrodes (7) sont respectivement appliquées à la surface du substrat de support (1') de telle manière qu'elles ne dépassent pas la surface de substrat de support (1').

14. Système d'électrodes selon l'une quelconque des revendications 1 à 13, **caractérisé en ce que** le substrat de support (1) est fabriqué à partir d'au moins un polymère biocompatible et comporte au moins par zones une substance active inhibant les réactions inflammatoires sur la surface de substrat de support cylindrique droite (1') tournée vers le faisceau de fibres nerveuses.

15. Système d'électrodes selon l'une quelconque des revendications 1 à 14, **caractérisé en ce qu'**au moins le détecteur et générateur de signaux (6), le deuxième détecteur de signaux prévu en option (6') et/ou une unité d'alimentation en énergie électrique sont montés hermétiquement, séparément du substrat de support (1) à l'intérieur d'un boîtier de type capsule ou sont des parties intégrantes du substrat de support (1).

16. Système d'électrodes selon l'une quelconque des revendications 1 à 15, **caractérisé en ce que** le substrat de support (1) contient un polymère biocompatible.

17. Système d'électrodes selon la revendication 16, **caractérisé en ce que** les premières et/ou deuxièmes bandes d'électrodes (5,8) disposent respectivement d'au moins une ouverture locale et **en ce que** les premières et/ou deuxièmes bandes d'électrodes (5,8) sont reliées à plat à la surface de substrat de support (1') de telle manière que le polymère traverse au moins une ouverture au moins en partie.

18. Système d'électrodes selon l'une quelconque des revendications 1 à 17, **caractérisé en ce qu'**à l'arrière de la surface de substrat de support (1') au moins deux surfaces d'électrodes de référence (12) sont appliquées au substrat de support (1).

19. Système d'électrodes selon l'une quelconque des revendications 1 à 18, **caractérisé en ce que** le substrat de support biocompatible (1) comporte dans la zone de la surface de substrat de support (1') cylindrique droite orientée vers le faisceau des fibres nerveuses des zones de bord s'opposant respectivement axialement sur lesquelles le substrat de support (1) dispose d'une épaisseur de substrat plus grande que dans la zone de substrat de support restante et **en ce que** les zones de bord disposent d'arêtes de bordure arrondies.

20. Système d'électrodes selon l'une quelconque des revendications 1 à 19, **caractérisé en ce que** le substrat de support biocompatible (1) comporte au moins une ouverture de fixation (14) traversant complètement le substrat de support dans une zone du substrat de support, qui ne peut pas faire l'objet d'une application sous la forme de manchette autour du faisceau de fibres nerveuses.

21. Système d'électrodes selon la revendication 20, **caractérisé en ce qu'**au moins une ouverture de fixation (14) est entourée au moins par zones d'un matériau métallique.

22. Système d'électrodes selon la revendication 16, **caractérisé en ce que** les premières et/ou deuxièmes bandes d'électrodes (5,8) comportent respectivement une plaque de base métallique (17) avec une face supérieure (18) et inférieure (19) plane, avec au moins un élément de structure (20) dépassant orthogonalement, localement la face supérieure (18), **en ce que** la surface plane (18) de la plaque de base métallique (17) est orientée parallèlement à la surface de substrat de support (1'),et **en ce que** la plaque de base métallique (17) est indirectement et/ou directement complètement entourée de polymère biocompatible à l'exception d'une première zone de surface (21) d'au moins un élément de structure (20) qui est orientée vers la surface de substrat de support (1') et ne dépasse pas celle-ci.

23. Système d'électrodes selon la revendication 22, **caractérisé en ce qu'**une couche d'agent adhésif (22) ou un système de couche d'agent adhésif (22') est incorporé au moins entre la face inférieure (19) de la plaque de base métallique (17) et le polymère biocompatible du substrat de support (1).

24. Système d'électrodes selon la revendication 22 ou 23, **caractérisé en ce que** la première zone de surface (21) d'au moins un élément de structure (20) ou un plan (E) attribué à la première zone de surface (21) est orienté parallèlement à la surface de substrat de support (1'), **en ce que** la première zone de surface (21) est disposée libre d'accès des côtés de la surface de substrat de support (1') et **en ce qu'**au moins un élément de structure (20) est relié en une pièce à la plaque de base métallique (17).

25. Système d'électrodes selon l'une quelconque des revendications 22 à 24, **caractérisé en ce qu'**il est prévu une pluralité d'éléments de structure (20) disposés sur la face supérieure (18) de la plaque de base métallique (17) selon un modèle géométrique (KO) et constitués de façon identique.

26. Système d'électrodes selon l'une quelconque des revendications 22 à 25, **caractérisé en ce qu'**au moins un élément de structure (20) est constitué en forme de colonne, de nervure, de manchon ou de moulure.

27. Système d'électrodes selon l'une quelconque des revendications 23 à 25, **caractérisé en ce qu'**au moins un élément de structure (20) comporte une extension longitudinale (LA) orientée orthogonalement à la face supérieure de la plaque de base métallique (17), le long de laquelle l'élément de structure (20) prévoit au moins une deuxième zone de surface (23), qui est orientée parallèlement à la face supérieure (18) de la plaque de base métallique (17) et sur laquelle est appliquée une couche d'agent adhésif (22) ou un système de couche d'agent adhésif (22') et **en ce que** la deuxième zone de surface (23) est disposée à distance de la première zone de surface (18) et est indirectement complètement entourée du polymère biocompatible.

28. Système d'électrodes selon l'une quelconque des revendications 1 à 27, **caractérisé en ce qu'**au moins dans une partie de zone du substrat de support (1) ne contenant pas la surface de substrat de support (1'), le substrat de support (1) est entouré par une manchette (M) .

29. Système d'électrodes selon la revendication 28 et 20 ou 21, **caractérisé en ce que** la manchette (1) dispose d'une face supérieure (Mo) et d'une face inférieure (Mu) de manchette qui sont reliées l'une à l'autre en une seule pièce de façon articulée et **en ce que** la face supérieure (Mo) et la face inférieure (Mu) de manchette comportent respectivement des ouvertures de fixation (14') qui sont disposées coïncidant avec les ouvertures de fixation (14) du substrat de support (1) à l'état de la manchette (M) englobant le substrat de support (1).

30. Système d'électrodes selon l'une quelconque des revendications 16 à 29, **caractérisé en ce que** le substrat de support (1) comporte une épaisseur de substrat orientée orthogonalement à la surface de substrat de support (1') et **en ce que** la plaque de base (17) est disposée de façon centrale par rapport à l'épaisseur de substrat.
